# EUROPÄISCHE PATENTSCHRIFT

(11) **EP 1 307 561 B1**
(45) Veröffentlichungstag und Bekanntmachung des Hinweises auf die Patenterteilung: **11.10.2006**
(21) Anmeldenummer: 01969445.4
(22) Anmeldetag: 20.07.2001
(51) Int. Cl.: C12N 15/29, C12N 15/82, C12N 15/11, C12N 5/10, C07K 14/415, C07K 16/16, A01H 5/00

(54) **NUCLEINSÄUREN, MIT DEREN HILFE PFLANZEN MIT VERÄNDERTEM METABOLIT-GEHALT ERZEUGT WERDEN KÖNNEN**
NUCLEIC ACIDS, WITH THE AID OF WHICH PLANTS HAVING AN ALTERED METABOLITE CONTENT CAN BE PRODUCED
ACIDES NUCLEIQUES A L'AIDE DESQUELS PEUVENT ETRE PRODUITS DES VEGETAUX A TENEUR EN METABOLITES MODIFIEE

(30) Priorität: 27.07.2000 DE 10036671
(43) Veröffentlichungstag der Anmeldung: 07.05.2003
(60) Teilanmeldung: 04008530.0
(73) Patentinhaber: Südzucker Aktiengesellschaft Mannheim/Ochsenfurt, 68165 Mannheim (DE)
(72) Erfinder: FROMMER, Wolf-Bernd, Carnegie Institution, Stanford, CA 94305-4101 (US); CATONI, Elisabetta, 70186 Stuttgart (DE); SCHWAB, Rebecca, 72074 Tübingen (DE); SCHUMACHER, Karin, 72070 Tübingen-Hagelloch (DE)
(74) Vertreter: Schrell, Andreas
(86) Internationale Anmeldenummer: PCT/EP2001/008379
(87) Internationale Veröffentlichungsnummer: WO 2002/010394

(56) Entgegenhaltungen:
- US-A- 5 981 219
- DATABASE EMBL [Online] AC AC011717; gene F19K16.14; prot_id AAG52250.1, 18. Oktober 1999 (1999-10-18) XP002192086
- DATABASE EMBL [Online] AC AL161746 gene T1008_50; protein_id CAB81917.1, 21. März 2000 (2000-03-21) XP002192087
- LALOI M: "Plant mitochondrial carriers: An overview." CMLS CELLULAR AND MOLECULAR LIFE SCIENCES, Bd. 56, Nr. 11-12, Dezember 1999 (1999-12), Seiten 918-944, XP002192083 ISSN: 1420-682X
- RICQUIER DANIEL ET AL: "Contribution to the identification and analysis of the mitochondrial uncoupling proteins." JOURNAL OF BIOENERGETICS AND BIOMEMBRANES, Bd. 31, Nr. 5, Oktober 1999 (1999-10), Seiten 407-418, XP002192084 ISSN: 0145-479X
- GILLISSEN ET AL: "A new family of high-affinity transporters for adenine, cytosine, and purine derivatives in arabidopsis" PLANT CELL, AMERICAN SOCIETY OF PLANT PHYSIOLOGISTS, ROCKVILLE, MD, US, Bd. 12, Nr. 2, Februar 2000 (2000-02), Seiten 291-300, XP002141279 ISSN: 1040-4651
- PALMIERI L ET AL: "Identification of the yeast ACR1 gene product as a succinate-fumarate transporter essential for growth on ethanol or acetate" FEBS LETTERS, ELSEVIER SCIENCE PUBLISHERS, AMSTERDAM, NL, Bd. 417, Nr. 1, 3. November 1997 (1997-11-03), Seiten 114-118, XP004261404 ISSN: 0014-5793 in der Anmeldung erwähnt
- CRABEEL MARJOLAINE ET AL: "The ARG11 gene of Saccharomyces cerevisiae encodes a mitochondrial integral membrane protein required for arginine biosynthesis." JOURNAL OF BIOLOGICAL CHEMISTRY, Bd. 271, Nr. 40, 1996, Seiten 25011-25018, XP002192085 ISSN: 0021-9258 in der Anmeldung erwähnt

### SEQUENCE LISTING

<110> Prof. Frommer, Wolf-Bernd
<120> Nucleinsäuren, mit deren Hilfe Pflanzen mit verändertem Metabolit-Gehalt erzeugt werden können.
<130> 16281
<140> unknown
   <141> 2000-07-24
<150> UNKNOWN
   <151> 2000-07-24
<160> 10
<170> PatentIn Ver. 2.1
<210> 1
   <211> 891
   <212> DNA
   <213> Arabidopsis thaliana
<400> 1
   atggatttct ggccggagtt tatggcgacc agctggggaa gagagttcgt cgccggtggt 60
   ttcggtggcg tcgccggcat catctccggc taccccttgg acaccctcag aattcgccag 120
   caacagagct cgaaatctgg atctgccttc tccattctcc ggcggatgct cgccattgag 180
   ggtccctcct ctctctacag aggcatggct gcgcctttgg cctccgtcac ttttcagaat 240
   gctatggtat tccagatata cgccatattc tctcgctctt ttgattcctc tgttcctctg 300
   gtagagcctc cttcctacag aggcgttgct cttggtggtg ttgccaccgg tgctgtacag 360
   agcctcttgc tcacccctgt cgagctcatc aagattcgtc tccagcttca gcagactaag 420
   tctggtccca tcaccttggc caagagcatc cttaggagac agggccttca ggggctttac 480
   agaggcctca ccatcaccgt gctccgagat gctcccgctc atggcctcta cttctggacc 540
   tatgagtacg tcagagaaag gcttcacccc ggctgcagaa agaccggaca agaaaacctc 600
   aggaccatgc tcgtggctgg tggccttgct ggagtggcca gctgggtcgc ttgttatcct 660
   cttgatgtcg tcaagaccag actccaacaa ggtcatgggg cttacgaggg cattgccgat 720
   tgttttcgca agagcgtcaa acaggaaggc tatacggttc tctggcgtgg cctcgggact 780
   gcagttgcca gggcctttgt ggtcaacggt gctatctttg ctgcttatga ggtagccttg 840
   cggtgtctat tcaatcaatc gccatcccca gatattgtca caggagattg a          891
<210> 2
   <211> 296
   <212> PRT
   <213> Arabidopsis thaliana
<400> 2
   Met Asp Phe Trp Pro Glu Phe Met Ala Thr Ser Trp Gly Arg Glu Phe
   1               5                   10                  15
   Val Ala Gly Gly Phe Gly Gly Val Ala Gly Ile Ile Ser Gly Tyr Pro
               20                  25                  30
   Leu Asp Thr Leu Arg Ile Arg Gln Gln Gln Ser Ser Lys Ser Gly Ser
           35                  40                  45
   Ala Phe Ser Ile Leu Arg Arg Met Leu Ala Ile Glu Gly Pro Ser Ser
       50                  55                  60
   Leu Tyr Arg Gly Met Ala Ala Pro Leu Ala Ser Val Thr Phe Gln Asn
   65                  70                  75                  80
   Ala Met Val Phe Gln Ile Tyr Ala Ile Phe Ser Arg Ser Phe Asp Ser
   85 90 95
   Ser Val Pro Leu Val Glu Pro Pro Ser Tyr Arg Gly Val Ala Leu Gly
   100 105 110
   Gly Val Ala Thr Gly Ala Val Gln Ser Leu Leu Leu Thr Pro Val Glu
   115 120 125
   Leu Ile Lys Ile Arg Leu Gln Leu Gln Gln Thr Lys Ser Gly Pro Ile
   130 135 140
   Thr Leu Ala Lys Ser Ile Leu Arg Arg Gln Gly Leu Gln Gly Leu Tyr
   145 150 155 160
   Arg Gly Leu Thr Ile Thr Val Leu Arg Asp Ala Pro Ala His Gly Leu
   165 170 175
   Tyr Phe Trp Thr Tyr Glu Tyr Val Arg Glu Arg Leu His Pro Gly Cys
   180 185 190
   Arg Lys Thr Gly Gln Glu Asn Leu Arg Thr Met Leu Val Ala Gly Gly
   195 200 205
   Leu Ala Gly Val Ala Ser Trp Val Ala Cys Tyr Pro Leu Asp Val Val
   210 215 220
   Lys Thr Arg Leu Gln Gln Gly His Gly Ala Tyr Glu Gly Ile Ala Asp
   225 230 235 240
   Cys Phe Arg Lys Ser Val Lys Gln Glu Gly Tyr Thr Val Leu Trp Arg
   245 250 255
   Gly Leu Gly Thr Ala Val Ala Arg Ala Phe Val Val Asn Gly Ala Ile
   260 265 270
   Phe Ala Ala Tyr Glu Val Ala Leu Arg Cys Leu Phe Asn Gln Ser Pro
   275 280 285
   Ser Pro Asp Ile Val Thr Gly Asp
   290 295
<210> 3
   <211> 930
   <212> DNA
   <213> Arabidopsis thaliana
<400> 3
   atggcgacga gaacggaatc gaagaagcag attccgccgt acatgaaagc agtctcaggc 60
   tcactaggcg gagtggtcga ggcttgttgt ctccaaccaa tcgacgtaat caaaacgcgt 120
   ctccagctag atcgcgtcgg cgcttacaaa ggaatcgctc actgtggttc gaaggtggtt 180
   cgcaccgaag gagttcgtgc tctctggaaa ggcttaacac cgttcgctac tcatctcacg 240
   cttaagtaca cgcttcggat gggatccaac gccatgtttc aaaccgcctt taaggattcc 300
   gagaccggaa aggtcagcaa tcgtggccgt tttctttccg gattcggtgc cggtgttctt 360
   gaagctctcg ccattgttac accctttgag gtggtgaaaa ttagacttca gcagcagaaa 420
   ggattgagtc ctgagctttt caagtacaaa ggaccaatac attgtgctag aaccatcgtg 480
   agagaagaaa gcatacttgg tttatggtca ggtgcagcac cgacggttat gcgaaacgga 540
   accaaccaag ctgtaatgtt cacagcgaaa aacgcgtttg acatactctt gtggaacaaa 600
   cacgaaggtg acggtaaaat cttgcagcca tggcagtcaa tgatctcagg gtttttagct 660
   ggaaccgcag gtccgttctg cacaggaccg tttgatgtgg tgaaaacgag gttgatggct 720
   cagagcagag acagtgaagg tgggattaga tataaaggga tggttcatgc cattagaacg 780
   atttatgcag aggaaggatt agtggcctta tggagagggt tattgccgag gctaatgagg 840
   attcctccag gacaagccat tatgtgggct gttgctgatc aagtcactgg tctttatgag 900
   atgagatatc ttcggaatgc tcctttatag                                  930
<210> 4
   <211> 309
   <212> PRT
   <213> Arabidopsis thaliana
<400> 4
   Met Ala Thr Arg Thr Glu Ser Lys Lys Gln Ile Pro Pro Tyr Met Lys
   1 5 10 15
   Ala Val Ser Gly Ser Leu Gly Gly Val Val Glu Ala Cys Cys Leu Gln
   20 25 30
   Pro Ile Asp Val Ile Lys Thr Arg Leu Gln Leu Asp Arg Val Gly Ala
   35 40 45
   Tyr Lys Gly Ile Ala His Cys Gly Ser Lys Val Val Arg Thr Glu Gly
   50 55 60
   Val Arg Ala Leu Trp Lys Gly Leu Thr Pro Phe Ala Thr His Leu Thr
   65 70 75 80
   Leu Lys Tyr Thr Leu Arg Met Gly Ser Asn Ala Met Phe Gln Thr Ala
   85 90 95
   Phe Lys Asp Ser Glu Thr Gly Lys Val Ser Asn Arg Gly Arg Phe Leu
   100 105 110
   Ser Gly Phe Gly Ala Gly Val Leu Glu Ala Leu Ala Ile Val Thr Pro
   115 120 125
   Phe Glu Val Val Lys Ile Arg Leu Gln Gln Gln Lys Gly Leu Ser Pro
   130 135 140
   Glu Leu Phe Lys Tyr Lys Gly Pro Ile His Cys Ala Arg Thr Ile Val
   145 150 155 160
   Arg Glu Glu Ser Ile Leu Gly Leu Trp Ser Gly Ala Ala Pro Thr Val
   165 170 175
   Met Arg Asn Gly Thr Asn Gln Ala Val Met Phe Thr Ala Lys Asn Ala
   180 185 190
   Phe Asp Ile Leu Leu Trp Asn Lys His Glu Gly Asp Gly Lys Ile Leu
   195 200 205
   Gln Pro Trp Gln Ser Met Ile Ser Gly Phe Leu Ala Gly Thr Ala Gly
   210 215 220
   Pro Phe Cys Thr Gly Pro Phe Asp Val Val Lys Thr Arg Leu Met Ala
   225 230 235 240
   Gln Ser Arg Asp Ser Glu Gly Gly Ile Arg Tyr Lys Gly Met Val His
   245 250 255
   Ala Ile Arg Thr Ile Tyr Ala Glu Glu Gly Leu Val Ala Leu Trp Arg
   260 265 270
   Gly Leu Leu Pro Arg Leu Met Arg Ile Pro Pro Gly Gln Ala Ile Met
           275                 280                 285
   Trp Ala Val Ala Asp Gln Val Thr Gly Leu Tyr Glu Met Arg Tyr Leu
       290                 295                 300
   Arg Asn Ala Pro Leu
   305
<210> 5
   <211> 33
   <212> DNA
   <213> Arabidopsis thaliana
<400> 5
   agcctcgaga tggatttctg gccggagttt atg 33
<210> 6
   <211> 30
   <212> DNA
   <213> Arabidopsis thaliana
<400> 6
   ttcggatcct caatctcctg tgacaatatc 30
<210> 7
   <211> 29
   <212> DNA
   <213> Arabidopsis thaliana
<400> 7
   acgctcgaga tggcgacgag aacggaatc 29
<210> 8
   <211> 32
   <212> DNA
   <213> Arabidopsis thaliana
<400> 8
   acggcggccg cctataaagg agcattccga ag 32
<210> 9
   <211> 33
   <212> DNA
   <213> Arabidopsis thaliana
<400> 9
   ttcggatcca tggatttctg gccggagttt atg 33
<210> 10
   <211> 31
   <212> DNA
   <213> Arabidopsis thaliana
<900> 10
   aatcccggga tctcctgtga caatatctgg g 31

### SEQUENCE LISTING

<110> Prof. Frommer, Wolf-Bernd
<120> Nucleinsäuren, mit deren Hilfe Pflanzen mit verändertem Metabolit-Gehalt erzeugt werden können.
<130> 16281
<140> unknown
   <141> 2000-07-24
<150> UNKNOWN
   <151> 2000-07-24
<160> 10
<170> PatentIn Ver. 2.1
<210> 1
   <211> 891
   <212> DNA
   <213> Arabidopsis thaliana
<400> 1
   atggatttct ggccggagtt tatggcgacc agctggggaa gagagttcgt cgccggtggt 60
   ttcggtggcg tcgccggcat catctccggc taccccttgg acaccctcag aattcgccag 120
   caacagagct cgaaatctgg atctgccttc tccattctcc ggcggatgct cgccattgag 180
   ggtccctcct ctctctacag aggcatggct gcgcctttgg cctccgtcac ttttcagaat 240
   gctatggtat tccagatata cgccatattc tctcgctctt ttgattcctc tgttcctctg 300
   gtagagcctc cttcctacag aggcgttgct cttggtggtg ttgccaccgg tgctgtacag 360
   agcctcttgc tcacccctgt cgagctcatc aagattcgtc tccagcttca gcagactaag 420
   tctggtccca tcaccttggc caagagcatc cttaggagac agggccttca ggggctttac 480
   agaggcctca ccatcaccgt gctccgagat gctcccgctc atggcctcta cttctggacc 540
   tatgagtacg tcagagaaag gcttcacccc ggctgcagaa agaccggaca agaaaacctc 600
   aggaccatgc tcgtggctgg tggccttgct ggagtggcca gctgggtcgc ttgttatcct 660
   cttgatgtcg tcaagaccag actccaacaa ggtcatgggg cttacgaggg cattgccgat 720
   tgttttcgca agagcgtcaa acaggaaggc tatacggttc tctggcgtgg cctcgggact 780
   gcagttgcca gggcctttgt ggtcaacggt gctatctttg ctgcttatga ggtagccttg 840
   cggtgtctat tcaatcaatc gccatcccca gatattgtca caggagattg a          891
<210> 2
   <211> 296
   <212> PRT
   <213> Arabidopsis thaliana
<400> 2
   Met Asp Phe Trp Pro Glu Phe Met Ala Thr Ser Trp Gly Arg Glu Phe
    1               5                  10                  15
   Val Ala Gly Gly Phe Gly Gly Val Ala Gly Ile Ile Ser Gly Tyr Pro
               20                  25                  30
   Leu Asp Thr Leu Arg Ile Arg Gln Gln Gln Ser Ser Lys Ser Gly Ser
           35                  40                  45
   Ala Phe Ser Ile Leu Arg Arg Met Leu Ala Ile Glu Gly Pro Ser Ser
       50                  55                  60
   Leu Tyr Arg Gly Met Ala Ala Pro Leu Ala Ser Val Thr Phe Gln Asn
   65                  70                  75                  80
   Ala Met Val Phe Gln Ile Tyr Ala Ile Phe Ser Arg Ser Phe Asp Ser
                   85                  90                  95
   Ser Val Pro Leu Val Glu Pro Pro Ser Tyr Arg Gly Val Ala Leu Gly
               100                 105                 110
   Gly Val Ala Thr Gly Ala Val Gln Ser Leu Leu Leu Thr Pro Val Glu
           115                 120                 125
   Leu Ile Lys Ile Arg Leu Gln Leu Gln Gln Thr Lys Ser Gly Pro Ile
       130                 135                 140
   Thr Leu Ala Lys Ser Ile Leu Arg Arg Gln Gly Leu Gln Gly Leu Tyr
   145                 150                 155                 160
   Arg Gly Leu Thr Ile Thr Val Leu Arg Asp Ala Pro Ala His Gly Leu
                   165                 170                 175
   Tyr Phe Trp Thr Tyr Glu Tyr Val Arg Glu Arg Leu His Pro Gly Cys
               180                 185                 190
   Arg Lys Thr Gly Gln Glu Asn Leu Arg Thr Met Leu Val Ala Gly Gly
           195                 200                 205
   Leu Ala Gly Val Ala Ser Trp Val Ala Cys Tyr Pro Leu Asp Val Val
       210                 215                 220
   Lys Thr Arg Leu Gln Gln Gly His Gly Ala Tyr Glu Gly Ile Ala Asp
   225                 230                 235                 240
   Cys Phe Arg Lys Ser Val Lys Gln Glu Gly Tyr Thr Val Leu Trp Arg
                   245                 250                 255
   Gly Leu Gly Thr Ala Val Ala Arg Ala Phe Val Val Asn Gly Ala Ile
               260                 265                 270
   Phe Ala Ala Tyr Glu Val Ala Leu Arg Cys Leu Phe Asn Gln Ser Pro
           275                 280                 285
   Ser Pro Asp Ile Val Thr Gly Asp
       290                 295
<210> 3
   <211> 930
   <212> DNA
   <213> Arabidopsis thaliana
<400> 3
   atggcgacga gaacggaatc gaagaagcag attccgccgt acatgaaagc agtctcaggc 60
   tcactaggcg gagtggtcga ggcttgttgt ctccaaccaa tcgacgtaat caaaacgcgt 120
   ctccagctag atcgcgtcgg cgcttacaaa ggaatcgctc actgtggttc gaaggtggtt 180
   cgcaccgaag gagttcgtgc tctctggaaa ggcttaacac cgttcgctac tcatctcacg 240
   cttaagtaca cgcttcggat gggatccaac gccatgtttc aaaccgcctt taaggattcc 300
   gagaccggaa aggtcagcaa tcgtggccgt tttctttccg gattcggtgc cggtgttctt 360
   gaagctctcg ccattgttac accctttgag gtggtgaaaa ttagacttca gcagcagaaa 420
   ggattgagtc ctgagctttt caagtacaaa ggaccaatac attgtgctag aaccatcgtg 480
   agagaagaaa gcatacttgg tttatggtca ggtgcagcac cgacggttat gcgaaacgga 540
   accaaccaag ctgtaatgtt cacagcgaaa aacgcgtttg acatactctt gtggaacaaa 600
   cacgaaggtg acggtaaaat cttgcagcca tggcagtcaa tgatctcagg gtttttagct 660
   ggaaccgcag gtccgttctg cacaggaccg tttgatgtgg tgaaaacgag gttgatggct 720
   cagagcagag acagtgaagg tgggattaga tataaaggga tggttcatgc cattagaacg 780
   atttatgcag aggaaggatt agtggcctta tggagagggt tattgccgag gctaatgagg 840
   attcctccag gacaagccat tatgtgggct gttgctgatc aagtcactgg tctttatgag 900
   atgagatatc ttcggaatgc tcctttatag 930
<210> 4
   <211> 309
   <212> PRT
   <213> Arabidopsis thaliana
<400> 4
   Met Ala Thr Arg Thr Glu Ser Lys Lys Gln Ile Pro Pro Tyr Met Lys
    1               5                  10                  15
   Ala Val Ser Gly Ser Leu Gly Gly Val Val Glu Ala Cys Cys Leu Gln
               20                  25                  30
   Pro Ile Asp Val Ile Lys Thr Arg Leu Gln Leu Asp Arg Val Gly Ala
           35                  40                  45
   Tyr Lys Gly Ile Ala His Cys Gly Ser Lys Val Val Arg Thr Glu Gly
       50                  55                  60
   Val Arg Ala Leu Trp Lys Gly Leu Thr Pro Phe Ala Thr His Leu Thr
   65                  70                  75                  80
   Leu Lys Tyr Thr Leu Arg Met Gly Ser Asn Ala Met Phe Gln Thr Ala
                   85                  90                  95
   Phe Lys Asp Ser Glu Thr Gly Lys Val Ser Asn Arg Gly Arg Phe Leu
               100                 105                 110
   Ser Gly Phe Gly Ala Gly Val Leu Glu Ala Leu Ala Ile Val Thr Pro
           115                 120                 125
   Phe Glu Val Val Lys Ile Arg Leu Gln Gln Gln Lys Gly Leu Ser Pro
       130                 135                 140
   Glu Leu Phe Lys Tyr Lys Gly Pro Ile His Cys Ala Arg Thr Ile Val
   145                 150                 155                 160
   Arg Glu Glu Ser Ile Leu Gly Leu Trp Ser Gly Ala Ala Pro Thr Val
                   165                 170                 175
   Met Arg Asn Gly Thr Asn Gln Ala Val Met Phe Thr Ala Lys Asn Ala
               180                 185                 190
   Phe Asp Ile Leu Leu Trp Asn Lys His Glu Gly Asp Gly Lys Ile Leu
           195                 200                 205
   Gln Pro Trp Gln Ser Met Ile Ser Gly Phe Leu Ala Gly Thr Ala Gly
       210                 215                 220
   Pro Phe Cys Thr Gly Pro Phe Asp Val Val Lys Thr Arg Leu Met Ala
   225                 230                 235                 240
   Gln Ser Arg Asp Ser Glu Gly Gly Ile Arg Tyr Lys Gly Met Val His
                   245                 250                 255
   Ala Ile Arg Thr Ile Tyr Ala Glu Glu Gly Leu Val Ala Leu Trp Arg
               260                 265                 270
   Gly Leu Leu Pro Arg Leu Met Arg Ile Pro Pro Gly Gln Ala Ile Met
                   275                 280                 285
   Trp Ala Val Ala Asp Gln Val Thr Gly Leu Tyr Glu Met Arg Tyr Leu
       290                 295                 300
   Arg Asn Ala Pro Leu
   305
<210> 5
   <211> 33
   <212> DNA
   <213> Arabidopsis thaliana
<400> 5
   agcctcgaga tggatttctg gccggagttt atg 33
<210> 6
   <211> 30
   <212> DNA
   <213> Arabidopsis thaliana
<400> 6
   ttcggatcct caatctcctg tgacaatatc 30
<210> 7
   <211> 29
   <212> DNA
   <213> Arabidopsis thaliana
<400> 7
   acgctcgaga tggcgacgag aacggaatc 29
<210> 8
   <211> 32
   <212> DNA
   <213> Arabidopsis thaliana
<400> 8
   acggcggccg cctataaagg agcattccga ag 32
<210> 9
   <211> 33
   <212> DNA
   <213> Arabidopsis thaliana
<400> 9
   ttcggatcca tggatttctg gccggagttt atg 33
<210> 10
   <211> 31
   <212> DNA
   <213> Arabidopsis thaliana
<400> 10
   aatcccggga tctcctgtga caatatctgg g 31

## Beschreibung

Die vorliegende Erfindung betrifft Verfahren zur Modifikation der Transporteigenschaften der inneren Mitochondrienmembran einer Pflanze, insbesondere eines Pflanzenteils.

Transporter spielen in der Funktion eines Organismus eine besondere Rolle. Sie entscheiden über die Aufnahme oder Abgabe eines Stoffes in eine oder aus einer Zelle beziehungsweise einem Organismus und steuern so den Transport und die Verteilung der Stoffe zwischen den Zellen. Auf der Ebene der intrazellulären Stoffverteilung kommt den Transportern der Organellen-Membranen eine wichtige regulatorische Funktion zu, da häufig einzelne Schritte eines Stoffwechselweges in verschiedenen Kompartimenten ablaufen und die Intermediate zwischen diesen Kompartimenten transportiert werden müssen.

Besonders wichtige Kompartimente stellen die auch als. Kraftwerke der Zelle bezeichneten Mitochondrien dar. Neben den Prozessen der Respiration und Photorespiration findet auch eine Vielzahl anabolischer und katabolischer Schritte verschiedener Stoffwechselwege in den Mitochondrien statt. Der Citratzyklus, der als zentrale Drehscheibe den Fluss der Kohlenstoffgrundgerüste für eine Vielzahl von Metaboliten kontrolliert, ist ebenfalls in den Mitochondrien lokalisiert. Es ist daher offensichtlich, dass eine Vielzahl von Metaboliten, wie zum Beispiel Pyruvat, Aminosäuren, Carbonsäuren und Fettsäuren, in die Mitochondrien hinein und aus ihnen heraus transportiert werden muss. Im Gegensatz zur äußeren Mitochondrienmembran, die für viele Substanzen frei passierbar ist, verfügt die innere Mitochondrienmembran über Transportsysteme hoher Spezifität. Eine Reihe dieser Transportsysteme umfasst Carrier, die nach dem Prinzip des Gegentausches (Antiport) arbeiten. Als Beispiele seien hier der Adenylnucleotid-Carrier, der exogenes ADP gegen internes ATP austauscht, der Dicarbonsäure-Carrier, der Malat gegen 2-Oxoglutarat tauscht und der Tricarbonsäure-Carrier, der Citrat gegen Malat austauscht, genannt. Andere Substanzen, wie Glutamat und Aspartat, werden ohne Gegentausch (Uniport) transportiert, während für einige andere Metabolite der genaue Transportmechanismus noch nicht bekannt ist. Die Transportsysteme der inneren Mitochondrienmembran ermöglichen den Metaboliten-Austausch und sind somit essentiell für die Integration des cytosolischen und des mitochondrialen Kompartiments. Die Mehrzahl der bislang vor allem in Hefe und in tierischen Systemen auf molekularer Ebene identifizierten Transporter der inneren Mitochondrienmembran gehören der "mitochondrial carrier"-Familie (MCF) an, die durch ihre konservierte Proteinstruktur und die Präsenz einer definierten Signatursequenz ("mitochondrial energy transfer sequence") gekennzeichnet ist (Palmieri, FEBS Letters, 346 (1994),48-54). Aufgrund phylogenetischer Analysen werden die MCF-Vertreter in insgesamt 17 Subfamilien unterteilt, wobei bislang nur für 5 Unterfamilien Substrate bekannt sind (Moualij et al., Yeast 13 (1997), 573-581).

Auch in höheren Pflanzen scheint eine große Familie kerncodierter Gene Vertreter dieser Proteinklasse zu codieren. Bislang wurden jedoch nur der ADP/ATP-Translokator bei einer Reihe von Pflanzenarten und der Malat/2-Oxoglutarat Translokator aus *Panicum* isoliert und charakterisiert. Interessanterweise ist auch der vom brittle-1 Gen von Mais codierte plastidäre Adenylat-Translokator ein MCF-Mitglied. Eine Analyse dieser Proteinfamilie in höheren Pflanzen kann daher nicht nur der Erforschung des mitochondrialen Transports, sondern auch des plastidären Transports dienen (Sullivan et al., Plant Cell, 3 (1991), 1337-48; Shannon et al., Plant Physiol., 117 (1998), 1235-52).

Durch die Analyse der Hefe-Mutanten *arg11* (Crabeel et al., J. Biol. Chem., 271 (1996), 25011-25018) und *acr1* (Palmieri et al., FEBS Lett., (1997), 114-118) wurden zwei MCF-Mitglieder identifiziert, die als Transporter für Arginin/Ornithin beziehungsweise für Succinat/Fumarat fungieren.

Biosynthese und Abbau der Aminosäure Arginin finden unter Mitwirkung mitochondrialer und cytosolischer Enzyme statt, so dass sowohl Arginin selbst als auch seine Vorstufe Ornithin die innere Mitochondrienmembran passieren müssen. Die *arg11*-Mutante der Hefe wurde als Arginin-auxotrophe Mutante isoliert, das heißt, sie ist nicht in der Lage, auf Medium ohne Arginin zu wachsen. Die Clonierung des *ARG11-*Gens und die biochemische Charakterisierung seines Genprodukts haben gezeigt, dass es sich um ein MCF-Mitglied handelt, welches sowohl Ornithin als auch Arginin transportieren kann. In höheren Pflanzen stellt die Aminosäure Arginin aufgrund ihres hohen Stickstoffgehalts eine bevorzugte Speicherform für Stickstoff dar. So liegen in den Keimblättern der Soja-Bohne 60% des in Form von freien Aminosäuren vorliegenden Stickstoffs und 18% des in Samen-Proteinen enthaltenen Stickstoffs als Arginin vor. Die Identifizierung intrazellulärer Transporter, insbesondere des mitochondrialen Arginin-Transporters, ist die Voraussetzung dafür, um gezielte Veränderungen des Proteingehalts und/oder der Proteinzusammensetzung von Nutzpflanzen durchführen zu können.

Der in den Mitochondrien stattfindende Citrat-Zyklus steht im Zentrum des Stoffwechsels aller Organismen. Die aus dem Citrat-Zyklus als biosynthetische Vorstufen entfernten Intermediate müssen durch sogenannte anaplerotische Reaktionen aufgefüllt werden. Der im Cytosol beziehungsweise in den Glyoxisomen ablaufende Glyoxylat-Zyklus stellt sowohl in Hefe als auch in Pflanzen die wichtigste anaplerotische Reaktion dar, da das aus dieser Reaktion hervorgehende Succinat zur Auffüllung des Citrat-Zyklus genutzt wird. Hierzu muss Succinat jedoch zunächst aus dem Cytosol in die Mitochondrien transportiert werden. In Hefe wird dieser Transport vom ACR1-Protein, bei dem es sich ebenfalls um ein MCF-Mitglied handelt, bewerkstelligt. In Abwesenheit von ACR1 können Hefezellen nicht auf Acetat oder Ethanol als einziger Kohlenstoffquelle wachsen, da das aus ihrem Abbau entstehende Succinat nicht in die Mitochondrien gelangt. In Pflanzen stellt der Transport von Succinat in die Mitochondrien einen entscheidenden Schritt bei der Mobilisierung des in Form von Fettsäuren gespeicherten Kohlenstoff dar. Das aus der β-Oxidation stammende Acetyl-CoA wird in den Glyoxisomen zu Succinat umgewandelt, welches dann in den Citrat-Zyklus eingeschleust werden kann. Die Effizienz und Geschwindigkeit, mit der die gespeicherte Energie mobilisiert werden kann, ist insbesondere bei der Keimung fettspeichernder Pflanzensamen von zentraler Bedeutung. Bei Pflanzen können Manipulationen des Transports von Succinat in die Mitochondrien daher zu einer gezielten Veränderung des Keimungsverhaltens eingesetzt werden.

Der vorliegenden Erfindung liegt das technische Problem zu Grunde, Verfahren und Mittel bereitzustellen, um Pflanzen im Hinblick auf einen modifizierten mitochondrialen Transport von Stoffwechselmetaboliten und gegebenenfalls auch einen modifizierten plastidären Transport von Stoffwechselprodukten gezielt verändern zu können. Die gezielte Veränderung des mitochondrialen Transports betrifft in bevorzugter Ausführung insbesondere den Arginin-Transport bei Nutzpflanzen, um deren Proteingehalt und/oder deren Proteinzusammensetzung gezielt zu modifizieren.

Erfindungsgemäß wurde das technische Problem gelöst, indem ein Gen, codierend einen Transporter der inneren Mitochondrienmembran, insbesondere ein pflanzliches MCF-Mitglied, nämlich den Arginin/Ornithin-Transporter, und das davon codierte Protein durch Komplementation der Hefe-Mutante *arg11* identifiziert und charakterisiert wurde. Dabei wurde erstmalig gezeigt, dass ein pflanzliches Protein der inneren Mitochondrienmembran auch in Hefe korrekt lokalisiert wird, das heißt seinen Bestimmungsort erreicht. Erfindungsgemäß wird eine, vorzugsweise isolierte und vollständig gereinigte Nucleinsäure verwendet, ausgewählt aus der Gruppe bestehend aus:
a) einer Nucleinsäure, die erhältlich ist durch Komplementierung MCF-defizienter Wirtszellen, die einen Defekt in dem Transportermolekül aufweisen, mit Nucleinsäuresequenzen einer pflanzlichen Genbank und durch Selektion MCF-positiver Wirtszellen, oder einem Fragment davon;
b) einer Nucleinsäure mit einer in SEQ ID Nr. 1 dargestellten Nucleotidsequenz oder einem Fragment davon;
c) einer Nucleinsäure mit einer Sequenz, die ein Protein mit einer in SEQ ID Nr. 2 dargestellten Sequenz codiert, oder einem Fragment davon;
d) einer Nucleinsäure, die zu einer Nucleinsäure nach a) bis c) komplementär ist, oder einem Fragment davon;
e) einer Nucleinsäure, die durch Substitution, Addition, Inversion und/oder Deletion einer oder mehrerer Basen einer Nucleinsäure nach a) bis d) erhältlich ist;
f) einer Nucleinsäure, die aufgrund der Degeneration des genetischen Codes mit einer Nucleinsäure nach a) bis e) oder einem Fragment davon hybridisiert; und
g) einem Fragment einer Nucleinsäure nach a), b), c), d), e) oder f), welches mindestens 10 Nucleotide, vorzugsweise mindestens 50 Nucleotide, besonders bevorzugt mindestens 200 Nucleotide umfasst und in einer Wirtszelle in Antisinnorientierung zu einem Promoter die Expression eines pflanzlichen Arginin/Ornithin-Transportermoleküls hemmen kann,
   in einem Verfahren zur Veränderung des Metabolitgehaltes von Pflanzen durch Modifikation der Transportereigenschaften der inneren Mitochondrienmembran einer Pflanze, eines Pflanzenteils oder einer Pflanzenzelle, umfassend
   - das Einbringen einer Nucleinsäure in eine Pflanzenzelle und
   - gegebenenfalls das Regenerieren einer ganzen Pflanze daraus,
sowie zur Hemmung der Expression eines endogenen MCF-Mitglieds der inneren Mitochondrienmembran pflanzlicher Zellen, zur Herstellung transgener Nutzplanzen mit veränderten mitochondrialen Transporteigenschaften und zur Identifizierung von Inhibitoren des durch Transporter der inneren Mitochondrienmembran vemittelten Stofftransports in pflanzlichen Mitochondrien.

Eine Nucleinsäure kann eine DNA-Sequenz, zum Beispiel cDNA oder genomische DNA-Sequenz, oder eine RNA-Sequenz, zum Beispiel mRNA-Sequenz sein.

Im Zusammenhang mit der vorliegenden Erfindung bedeuten die Begriffe "MCF-Mitglied" und "MCF-Protein" ein Protein mit MCF-Aktivität, das heißt ein Protein, das an dem Transport von Metaboliten durch die innere Mitochondrienmembran, gegebenenfalls auch durch die innere Plastidenmembran, beteiligt ist und das die typischen Merkmale dieser ProteinFamilie aufweist. Bei den typischen Merkmalen handelt es sich zum einen um die Primärstruktur, die aus einer dreifachen Wiederholung einer circa 100 Aminosäuren umfassenden Domäne mit je zwei Membranspannen besteht, sowie um das Vorliegen von bis zu drei Wiederholungen der sogenannten "mitochondrial energy transfers"-Signatur (P-X-(D/E)-X-(LIVAT)-(RK)-X-(LRH)-(LIVMFY). Die Begriffe "MCF-Mitglied" und "MCF-Protein" umfassen im Zusammenhang mit der vorliegenden Erfindung insbesondere Transportermoleküle für Arginin/Ornithin. Zum Nachweis der Transporter-Aktivität kann beispielsweise das von Palmieri et al. (FEBS Letters, 417 (1997), 447) beschriebene Verfahren eingesetzt werden.

Im Zusammenhang mit der vorliegenden Erfindung bedeuten "MCF-defiziente Zellen" oder "MCF-defiziente Wirtszellen" Zellen, die aufgrund eines oder mehrerer genetischer Defekte negativ veränderte mitochondriale Transporteigenschaften aufweisen, die zu einem negativ selektierbaren Phänotyp führen. Bei MCF-defizienten Pflanzenzellen kann gegebenenfalls auch der plastidäre Transport negativ verändert sein.

Im Zusammenhang mit der vorliegenden Erfindung bedeuten "MCF-positive Zellen oder Wirtszellen" daher Zellen, die entweder natürlicherweise eine ein MCF-Mitglied codierende Nucleinsäure enthalten, oder Zellen, die aufgrund einer Komplementation MCFnegativer Zellen eine Nucleinsäure enthalten, die den/die dem negativ veränderten mitochondrialen Transport zu Grunde liegenden genetischen Defekts/Defekte zumindest teilweise aufhebt, und die daher einen positiv selektierbaren Phänotyp zeigen.

Der in der vorliegenden Erfindung verwendete Begriff "Komplementation" bezeichnet eine sich im Phänotyp eines Organismus widerspiegelnde Kompensation eines genetischen Funktionsdefektes unter Beibehaltung der dem Defekt zu Grunde liegenden Mutation(en). Eine Komplementation im Zusammenhang mit der Erfindung liegt beispielsweise vor, wenn ein genetischer Defekt in einem MCF-Gen (zum Beispiel im ARG11-Gen von *Saccharomyces cerevisiae*) durch die Anwesenheit eines gleichartigen, intakten Gens (zum Beispiel des AtARG11-Gens aus *Arabidopsis thaliana*) aufgehoben wird, wobei das intakte Gen die Funktion des defekten MCF-Gens übernimmt.

MCF-defiziente Zellen oder Wirtszellen können zur Isolierung und Identifizierung von Nucleinsäuren verwendet werden, die ein MCF-Mitglied, insbesondere ein Transportermolekül für Arginin/Ornithin codieren. Bevorzugte MCF-defiziente Wirtszellen sind eukaryontische Zellen, beispielsweise pflanzliche oder tierische Zellen, vorzugsweise Hefezellen. Die Identifizierung eines MCF-Mitglieds erfolgt durch Komplementation spezifischer Mutationen in MCF-defizienten Wirtszellen, insbesondere spezifischen Mutanten der Hefe *Sacchoramyces cerevisiae.* Dabei werden Nucleinsäuresequenzen einer pflanzlichen oder tierischen Genbank, zum Beispiel einer cDNA-Bank oder genomischen Bank, in MCF-defiziente Wirtszellen transformiert und anschließend erfolgt eine Selektion auf MCF-positive Wirtszellen.

Zur Isolierung eines Gens, das ein spezifisches Transportermolekül codiert, sind geeignete Hefe-Mutanten erforderlich, die aufgrund eines Defektes in diesem Transportermolekül nicht in der Lage sind, eine bestimmte Substanz in die Mitochondrien zu transportieren. Von Delforge et al. (Eur. J. Biochem., 57 (1975), 231) ist beispielsweise die Mutante *arg11* (Stamm MG409) beschrieben, die nur in Medien wachsen kann, die die Aminosäure Arginin enthalten. Zur Durchführung der Komplementation mit pflanzlichen oder tierischen Genen wurde in dieser Mutante zusätzlich das URA3-Gen zerstört, wobei eine Uracil-Auxotrophie erzeugt wurde (MG409ura3⁻, 1c1636d). Das heißt, dass diese Mutante zum Wachstum außerdem Uracil benötigt.

Zur Bereitstellung einer erfindungsgemäß verwendeten Nucleinsäure wird eine geeignete Hefemutante, beispielsweise die *arg11*/*ura3*-Mutante, mit zur Verwendung in Hefe geeigneten Expressions-Plasmiden transformiert, die cDNA-Fragmente aus einer pflanzlichen oder tierischen cDNA-Genbank enthalten. Sequenzen, die pflanzliche oder tierische mitochondriale Transportermoleküle codieren, lassen sich durch Selektion von Transformanten identifizieren, die aufgrund der Expression dieser pflanzlichen oder tierischen cDNA-Sequenzen auf Medium ohne Arginin wachsen können. Erfindungsgemäß können auch Nucleinsäuren, die Proteine mit Sequenzhomologie zu bereits bekannten mitochondrialen Transportermolekülen codieren, in geeignete Hefe-Expressionsplas-mide cloniert und auf ihre Fähigkeit zur Komplementation spezifischer Mutationen getestet werden. Die vorstehend beschriebenen Verfahren können zur Identifizierung und/oder Isolierung von Nucleinsäuren, die ein Protein mit der Aktivität eines MCF-Proteins codieren, eingesetzt werden, wobei MCF-defiziente Wirtszellen mit Nucleinsäuresequenzen einer pflanzlichen oder tierischen Genbank transformiert, MCF-positive Wirtszellen selektiert und die in den positiven Wirtszellen vorhandenen MCF-codierenden Nucleinsäuren mittels herkömmlicher Verfahren isoliert und/oder identifiziert werden.

Unter Verwendung der *arg11*/*ura3*-Hefemutante und geeigneter Expressions-Plasmide, die den Promoter der Protonen-ATPase PMA1 aus Hefe enthalten, wurden cDNA-Clone aus *Arabidopsis thaliana* isoliert, die eine Komplementation der Hefe-Mutation bewirken; Eine Analyse unter Verwendung von Sequenzierungs- und Restriktionsverfahren sowie weiterer biochemisch-molekularbiologischer Verfahren zeigte, dass diese erfindungsgemäß isolierten *Arabidopsis thali*ana-cDNA-Fragmente pflanzliche MCF-Mitglieder codieren. Die Nucleinsäuresequenz eines dieser cDNA-Clone ist in SEQ ID Nr. 1 gezeigt und die entsprechende Aminosäuresequenz dieses cDNA-Clons ist in SEQ ID Nr. 2 beschrieben.

Die Erfindung umfasst auch die Verwendung modifizierter Nucleinsäuren, die beispielsweise durch Substitution, Addition, Inversion und/oder Deletion einer oder mehrerer Basen einer erfindungsgemäßen Nucleinsäure, insbesondere innerhalb der codierenden Sequenz einer Nucleinsäure, erhältlich sind, das heißt also auch Nucleinsäuren, die als Mutanten, Derivate oder funktionellen Äquivalente der verwendeten Nucleinsäure bezeichnet werden können. Solche Manipulationen der Sequenzen werden beispielsweise durchgeführt, um die von einer Nucleinsäure codierte Aminosäuresequenz gezielt zu verändern, beispielsweise zur Veränderung der Spezifität eines mitochondrialen Transporters. Nucleinsäuren, die veränderte mitochondriale Transporter codieren, können unter anderem zur Transformation landwirtschaftlich genutzter Pflanzen verwendet werden, um transgene Pflanzen herzustellen. Gezielte Sequenzveränderungen können auch dem Ziel dienen; innerhalb der Nucleinsäuresequenz geeignete Restriktionsschnittstellen bereitzustellen oder nicht erforderliche Nucleinsäuresequenzen oder Restriktionsschnittstellen zu entfernen. Dabei werden die verwendeten Nucleinsäuren in Plasmiden inseriert und mittels Standardverfahren der Mikrobiologie/Molekularbiologie einer Mutagenese oder einer Sequenzveränderung durch Rekombination unterzogen. Zur Erzeugung von Insertionen, Deletionen oder Substitutionen, wie Transitionen und Transversionen, sind beispielsweise Verfahren zur in vitro-Mutagenese, "primer repair"-Verfahren sowie Restriktions- und/oder Ligationsverfahren geeignet (vgl. Sambrook et al., 1989, Molecular Cloning: A Laboratory Manual, 2. Auflage (1989), Cold Spring Harbor Laboratory Press, NY, USA). Sequenzveränderungen lassen sich auch durch Anlagerung natürlicher oder synthetischer Nucleinsäuresequenzen erreichen. Beispiele für synthetische Nucleinsäuresequenzen sind Adaptoren oder Linker, die u.a. auch zur Verknüpfung von Nucleinsäure-Fragmenten an diese Fragmente angefügt werden.

Die vorliegende Erfindung betrifft auch die Verwendung von Nucleinsäuren, die mit einer der vorstehend beschriebenen Nucleinsäuren nach a) bis e) hybridisieren. Der im Zusammenhang mit der vorliegenden Erfindung verwendete Ausdruck "Nucleinsäure, die mit einer Nucleinsäure nach a) bis e) hybridisiert" bedeutet eine Nucleinsäure, die unter mäßig stringenten Bedingungen mit einer Nucleinsäure nach a) bis e) hybridisiert. Beispielsweise kann die Hybridisierung mit einer radioaktiven Gensonde in einer Hybridisierungslösung (25% Formamid; 5 x SSPE; 0,1% SDS; 5 x Denhardt-Lösung; 50 µg/ml Heringsperma-DNA; bezüglich Zusammensetzung der Einzelkomponenten vgl. Sambrook et al., Molecular Cloning: A Laboratory Manual, 2. Auflage (1989), Cold Spring Harbor Laboratory Press, NY, USA) 20 Stunden bei 37°C erfolgen. Anschließend wird unspezifisch gebundene Sonde durch mehrfaches Waschen der Filter in 2 x SSC/0,1% SDS bei 42°C entfernt. Vorzugsweise werden die Filter mit 0,5 x SSC/0,1% SDS, besonders bevorzugt mit 0,1 x SSC/0,1% SDS bei 42°C gewaschen.

Die vorliegende Erfindung betrifft ebenfalls die Verwendung von Nucleinsäuren, die ein Polypeptid oder Protein mit MCF-Aktivität codieren, dessen Sequenz mindestens 40%, vorzugsweise mindestens 60%, besonders bevorzugt mindestens 80% Homologie zu einem Polypeptid oder Protein aufweist, das von einer Nucleinsäure mit einer in SEQ ID Nr. 1 dargestellten Sequenz codiert wird.

Im Zusammenhang mit der Erfindung bezieht sich der Ausdruck "mindestens 40%, vorzugsweise mindestens 60%, besonders bevorzugt mindestens 80% Homologie" auf eine Sequenzübereinstimmung auf der Aminosäuresequenz-Ebene, die mit Hilfe bekannter Verfahren, zum Beispiel computergestützter Sequenzvergleiche (Basic local alignment search tool, S. F. Altschul et al., J. Mol. Biol. 215 (1990), 403-410), bestimmt werden kann.

Der dem Fachmann bekannte Ausdruck "Homologie" bezeichnet den Grad der Verwandtschaft zwischen zwei oder mehreren Polypeptid-Molekülen, der durch die Übereinstimmung zwischen den Sequenzen bestimmt wird, wobei Übereinstimmung sowohl eine identische Übereinstimmung als auch einen konservativen Aminosäure-Austausch bedeuten kann. Der Prozentsatz der "Homologie" ergibt sich aus dem Prozentsatz übereinstimmender Bereiche in zwei oder mehr Sequenzen unter Berücksichtigung von Lücken oder anderen Sequenzbesonderheiten.

Der Begriff "konservativer Aminosäure-Austausch" bedeutet den Austausch eines Aminosäurerestes gegen einen anderen Aminosäurerest, wobei dieser Austausch nicht zu einer Änderung der Polarität oder Ladung an der Position der ausgetauschten Aminosäure führt. Ein Beispiel für einen konservativen Aminosäure-Austausch ist der Austausch eines unpolaren Aminosäurerestes gegen einen anderen unpolaren Aminosäurerest. Konservative Aminosäure-Austausche im Zusammenhang mit der Erfindung umfassen beispielsweise:
G=A=S, I=V=L=M, D=E, N=Q, K=R, Y=F, S=T
   G=A=I=V=L=M=Y=F=W=P=S=T.

Die Homologie zwischen miteinander verwandten Polypeptid-Molekülen kann mit Hilfe bekannter Verfahren bestimmt werden. In der Regel werden spezielle Computerprogramme mit den besonderen Anforderungen Rechnung tragenden Algorithmen eingesetzt. Bevorzugte Verfahren zur Bestimmung der Homologie erzeugen zunächst die größte Übereinstimmung zwischen den untersuchten Sequenzen. Computerprogramme zur Bestimmung der Homologie zwischen zwei Sequenzen umfassen, ohne jedoch darauf beschränkt zu sein, das GCG-Programmpaket, einschließlich GAP (Devereux, J., et al., Nucleic Acids Research, 12 (12) (1984), 387; Genetics Computer Group University of Wisconsin, Madison (WI)); BLASTP, BLASTN und FASTA (S. Altschul et al., J. Mol. Biol. 215 (1990), 403-410). Das BLASTX Programm kann vom National Centre for Biotechnology Information (NCBI) und aus weiteren Quellen bezogen werden (Altschul S., et al., BLAST Handbuch, NCB NLM NIH Bethesda MD 20894; Altschul, S., et al., J. Mol.Biol 215 (1990), 403-410). Auch der bekannte Smith Waterman-Algorithmus kann zur Bestimmung der Homologie verwendet werden.

Bevorzugte Parameter für den Sequenz-Vergleich umfassen beispielsweise:

| | |
|---|---|
| Algorithmus: | Needleman und Wunsch, J. Mol. Biol 48 (1970),443-453; |
| Vergleichsmatrix: | BLOSUM 62 von Henikoff und Henikoff, Proc. Natl. Acad. Sci. USA, 89 (1992), 10915-10919; |
| Lücken-Wert (Gap Penalty): | 12 |
| Lückenlängen-Wert (Gap Length Penalty): | 4 |
| Schwellenwert der Ähnlichkeit: | 0 |

Auch das GAP-Programm eignet sich zur Verwendung der vorstehend beschriebenen Parameter. Bei den vorstehend beschriebenen Parameter handelt es sich um Standardparameter (default parameters) für Aminosäuresequenz-Vergleiche.

Darüber hinaus lassen sich weitere Algorithmen, Lücken-Öffnungs-Werte (gap opening penalties), Lückenausdehnungs-Werte (gap extension penalties) und Vergleichsmatrizen einschließlich der im Programm-Handbuch, Wisconsin-Paket, Version 9 (September 1997) beschriebenen verwenden. Die Auswahl der Programme hängt sowohl von dem durchzuführenden Vergleich als auch davon ab, ob der Vergleich zwischen Sequenzpaaren durchgeführt wird, wobei GAP oder Best Fit bevorzugt sind, oder zwischen einer Sequenz und einer umfangreichen Sequenz-Datenbank, wobei FASTA oder BLAST bevorzugt sind.

Ein, vorzugsweise isoliertes und vollständig gereinigtes, Protein, kann durch Expression einer erfindungsgemäß verwendeten Nucleinsäure oder eines Fragments davon in einer Wirtszelle erhältlich sein. Vorzugs-weise besitzt das Protein die gleichen Transporteigenschaften wie das Protein, das von einer Nucleinsäure mit einer in SEQ ID Nr. 1 dargestellten Sequenz codiert wird. Zum Nachweis der Aktivität eines solchen Proteins können beispielsweise Aufnahmeexperimente durchgeführt werden, wie nachfolgend im Ausführungsbeispiel beschrieben.

Auch isolierte und vollständig gereinigte monoclonale oder poly-clonale Antikörper oder deren Fragmente können mit einem erfindungsgemäß verwendeten Protein reagieren.

Ein Konstrukt, kann eine erfindungsgemäß verwendete Nucleinsäure und/oder ein Fragment davon unter der Kontrolle von Expressions- Regulationselementen enthalten. Im Zusammenhang mit der vorliegenden Erfindung bedeutet ein "Konstrukt", das hier auch als Vektor bezeichnet sein kann, die Kombination einer erfindungsgemäßen Nucleinsäure oder eines Fragments davon mit mindestens einem Nucleinsäure-Zusatzelement, beispielsweise einem Regulationselement. Beispiele für solche Regulationselemente sind konstitutive oder induzierbare Promotoren, wie der *E.coli*-Promotor araBAD (Carra und Schlief, EMBO J., 12 (1993), 35-44) zur Expression in Bakterien, der Hefepromotor PMA1 (Rentsch et al., FEBS Lett., 370 (1995), 264-268) zur Expression in Pilz-Systemen und der virale CaMV35S Promotor (Pietrzak et al., Nucl. Acids Res., 14 (1986), 5857-5868) zur Expression in Pflanzen. Ferner kann die Nucleinsäure oder das Fragment mit einem Transkriptions-Terminationssignal versehen werden. Derartige Elemente sind bereits beschrieben worden (vgl. zum Beispiel Gielen et al., EMBO J., 8 (1984), 23-29) . Die Transkriptionsstart- und -terminationsbereiche können nativ (homolog) als auch fremdartig (heterolog) zum Wirtsorganismus sein. Die Sequenz der Transkriptionsstart- und -terminationsregionen kann synthetischen oder natürlichen Ursprungs sein oder eine Mischung aus synthetischen und natürlichen Bestandteilen enthalten. Das Konstrukt kann ein Plasmid sein.

Die Nucleinsäure oder das Fragment kann in dem Konstrukt, insbesondere einem Plasmid, sowohl in Antisinnorientierung als auch in Sinnorientierung zu dem/den regulatorischen Element(en) vorliegen. Liegt die Nucleinsäure oder das Fragment beispielsweise in Sinnorientierung zu dem regulatorischen Element, beispielsweise einem Promotor, vor, kann sie/es, insbesondere nach Transformation und Integration in höherer Kopiezahl in das Genom, durch Cosuppressionseffekte die Aktivität des endogenen Transporters der inneren Mitochondrienmembran hemmen oder reduzieren. Wenn die Nucleinsäure oder das Fragment in dem Konstrukt in Antisinnorientierung zu dem regulatorischen Element vorliegt, kann das Konstrukt beispielsweise in das Genom einer pflanzlichen Wirtszelle eingeführt werden und nach seiner Transkription zur Unterdrückung der Bildung der pflanzeneigenen mitochondrialen TransporterMoleküle führen.

Ein Konstrukt kann eine erfindungsgemäß verwendete Nucleinsäure oder ein Fragment davon in Antisinn-orientierung zu einem Promotor umfassen, wobei in einer das Konstrukt enthaltenden Wirts-zelle die Expression eines mitochondrialen Transportermoleküls oder gegebenenfalls die Expression eines plastidären Transportermoleküls gehemmt wird. Dabei umfasst das Nucleinsäure-Fragment mindestens 10 Nucleotide, vorzugsweise mindestens 50 Nucleotide, besonders bevorzugt mindestens 200 Nucleotide. Das Konstrukt, das eine erfindungsgemäße Nucleinsäure oder ein Fragment davon enthält, kann in eine Wirtszelle eingebracht und dort in eine nichttranslatierbare RNA (Antisinn-RNA) transkribiert werden, die durch Bindung an ein endogenes Gen für einen mitochondrialen Transporter oder an die daraus transkribierte mRNA die Expression dieses endogenen Gens hemmen kann.

Ein Konstrukt kann eine erfindungsgemäß verwendete, Nucleinsäure oder ein erfindungsgemäß verwendetes Nucleinsäure-Fragment in Sinnorientierung zu einem regulatorischen Element, zum Beispiel einem Promoter, enthalten, dem sich eine weitere, gleiche oder andere erfindungsgemäß verwendete Nucleinsäure oder ein weiteres, gleiches oder anderes erfindungsgemäß verwendetes Nucleinsäure-Fragment in Antisinnorientierung dazu anschließt. Diese Anordnung ermöglicht die Ausbildung einer Doppelstrang-RNA, die den Abbau der endogenen RNA induzieren kann (Chuang und Meyerowitz, Proc. Natl. Acad. Sci. USA, 97 (2000),4985-90; Sijen und Kooter, Bioessays, 22, (2000), 520-531).

Das Plasmid kann ein Replikationssignal für *E. coli* und/oder Hefe und ein Marker-Gen enthalten, das eine positive Selektion der mit dem Plasmid transformierten Wirtszellen erlaubt. Wird das Plasmid in eine pflanzliche Wirtszelle eingeführt, so können je nach Einführungsverfahren weitere Sequenzen erforderlich sein, die dem Fachmann bekannt sind. Ist das Plasmid beispielsweise ein Derivat des Ti- oder Ri-Plasmids, so muss die einzuführende Nucleinsäure oder das einzuführende Fragment davon von T-DNA-Sequenzen flankiert sein, die die Inte-gration der Nucleinsäure oder des Fragments davon in das pflanzliche Genom ermöglichen. Die Verwendung von T-DNA zur Transformation von Pflanzenzellen ist intensiv untersucht worden und beispielsweise in EP 120 516; Hoekema, The Binary Plant Vector System, Kapitel V (1985), Offset-drukkerij Kanters B.V. Ablasserdam; Fraley et al., Crit. Rev. Plant Sci., 4 (1985), 1-46, und An et al., EMBO J., 4 (1985), 277-287 beschrieben. Ist die eingeführte Nucleinsäure oder das Fragment davon einmal im Genom integriert, so ist sie/es dort in der Regel stabil und wird auch in der Nachkommenschaft der ursprünglich transformierten Zelle erhalten.

Die im Genom integrierte Sequenz kann ebenfalls einen Selektionsmarker enthalten, der beispielsweise den transformierten Pflanzenzellen Resistenz gegenüber einem Biozid oder einem Antibiotikum, wie Kanamycin, G418, Bleomycin, Hygromycin oder Phosphinotricin, verleiht. Der verwendete Marker gestattet die Selektion transformierter Zellen gegenüber Zellen, in denen die transformierte DNA nicht vorhanden ist.

Eine Wirtszelle die eine erfindungsgemäß verwendete Nucleinsäure, insbesondere eine Nucleinsäure mit einer in SEQ ID Nr. 1 dargestellten Sequenz, oder ein Fragment davon oder ein eine erfindungsgemäße Nucleinsäure oder ein Fragment davon umfassendes Konstrukt enthalten. Dabei kann es sich um ein Bakterium oder eine Hefe-, Insekten-, Säuger- oder Pflanzenzelle handeln.

Eine transgene Pflanze kann in mindestens einer ihrer Zellen eine erfindungsgemäß verwendete Nucleinsäure, die ein Protein mit MCF-Aktivität codiert, oder ein Fragment davon oder ein Konstrukt enthalten. Bei transgenen Pflanzen kann es sich um Pflanzen der verschiedensten Arten, Gattungen, Familien, Ordnungen und Klassen handeln, das heißt sowohl monocotyle als auch dicotyle Pflanzen, ebenso wie Algen, Moose, Farne oder Gymnospermae. Transgene Pflanzen können auch Kalli, Pflanzenzellkulturen, sowie Teile, Organe, Gewebe, Ernte- oder Vermehrungsmaterialien dieser Pflanzen umfassen. Dabei handelt es sich bei den transgenen Pflanzen insbesondere um Tabak-, Kartoffel-, Tomaten-, Zuckerrüben-, Sojabohnen-; Kaffee-, Erbsen-, Bohnen-, Baumwoll-, Reis- oder Maispflanzen.

Im Zusammenhang mit der vorliegenden Erfindung bedeutet der Ausdruck "in mindestens einer ihrer Zellen", dass eine transgene Pflanze mindestens eine Zelle, vorzugsweise jedoch eine Vielzahl von Zellen enthält, die eine oder mehrere stabil integrierte erfindungsgemäße Nucleinsäure oder ein Fragment davon oder ein erfindungsgemäßes Konstrukt enthalten. Die Nucleinsäure oder ein Fragment davon oder ein erfindungsgemäßes Konstrukt kann sowohl im Zellkern als auch im mitochondrialen oder plastidären Genom integriert sein. Vorzugsweise ist die Nucleinsäure, das Fragment oder das Konstrukt an einer Stelle der Zelle integriert, die nicht ihrer/seiner natürlichen Position entspricht, oder in einer Kopienzahl und/oder Orientierung integriert, die nicht der natürlicherweise vorkommenden Kopienzahl und/oder Orientierung entspricht.

Zur Herstellung einer transgenen, vorzugsweise fertilen Pflanze kann ein Verfahren eingesetzt werden, das die folgenden Schritte umfasst:
- Einbringen einer erfindungsgemäß verwendeten Nucleinsäure, insbesondere einer Nucleinsäure mit einer in SEQ ID Nr. 1 dargestellten Sequenz oder eines Fragments davon oder eines die Nucleinsäure oder ein Fragment davon enthaltenden Konstrukts in eine Pflanzenzelle; und
- Regenerieren einer, vorzugsweise fertilen Pflanze aus der transformierten Pflanzenzelle, wobei mindestens eine Zelle dieser Pflanze transgen ist, das heißt eine stabil integrierte Nucleinsäure der vorliegenden Erfindung enthält und vorzugsweise exprimiert, das heißt die integrierteNucleinsäure zu RNA transkribiert. Vorzugsweise weist die erhaltene Pflanze in mindestens einem ihrer Mitochondrien eine modifizierte Aktivität eines Transporters der inneren Mitochondrienmembran auf.

Für das Einbringen einer Nucleinsäure in eine Pflanzenzelle stehen eine Vielzahl von Verfahren zur Verfügung. Bei den meisten der Verfahren ist es erforderlich, dass die einzuführende Nucleinsäure in einem Konstrukt, wie einem Vektor, vorliegt. Vektoren, beispielsweise Plasmide, Cosmide, Viren, Bakteriophagen, Shuttle-Vektoren und ähnliche sind bekannt. Vektoren umfassen oft Funktionseinheiten, die der Stabilisierung des Vektors in einer Wirtszelle dienen und seine Replikation darin ermöglichen. Außerdem können Vektoren Regulationselemente enthalten, mit denen die Nucleinsäure funktionell verbunden ist und die die Expression der Nucleinsäure ermöglichen.

Neben der Transformation mit Hilfe von Agrobakterien, *beispielsweise Agrobacterium tumefaciens,* stehen noch zahlreiche weitere Verfahren zur Verfügung. Diese Verfahren umfassen die Fusion von Protoplasten, die Mikroinjektion von DNA, die Elektroporation sowie biolistische Verfahren und Virusinfektions-Verfahren. Anders als bei der Transformation mit Hilfe von Agrobakterien, werden bei der Injektion und Elektroporation an sich keine speziellen Anforderungen an den Vektor gestellt. Es können einfache Plasmide wie zum Beispiel pUC-Derivate verwendet werden. Sollen aber aus derartig transformierten Zellen ganze Pflanzen regeneriert werden, ist die Anwesenheit eines selektierbaren Markergens vorteilhaft.

Aus den transformierten Pflanzenzellen können dann in einem geeigneten Medium, das gegebenenfalls Antibiotika oder Biozide zur Selektion enthält, wieder ganze Pflanzen regeneriert werden. Die so erhaltenen Pflanzen können dann auf Anwesenheit der eingeführten DNA getestet werden. Die transformierten Zellen wachsen innerhalb der Pflanzen in der üblichen Weise (siehe auch McCormick et al., Plant Cell Reports, 5 (1986), 81-84). Diese Pflanzen können wie üblich angezogen werden und mit Pflanzen, die die gleiche transformierte Erbanlage oder andere Erbanlagen besitzen, gekreuzt werden. Die daraus entstehenden hybriden Individuen weisen die entsprechenden phänotypischen Eigenschaften auf.

Die vorliegende Erfindung umfasst auch ein Verfahren zur Modifikation der Eigenschaften des mitochondrialen und/oder plastidären Transports einer Pflanze, insbesondere zur Modifikation des mitochondrialen Arginin/Ornithin-Transports in einer Pflanzenzelle, einem Pflanzengewebe, einem Pflänzenorgan und/oder einer ganzen Pflanze, wobei eine erfindungsgemäße Nucleinsäure, insbesondere eine Nucleinsäure mit einer in SEQ ID Nr. 1 dargestellten Sequenz oder ein Fragment davon in eine Pflanzenzelle und/oder eine Pflanze eingebracht und anschließend eine ganze Pflanze regeneriert wird, wobei in mindestens einer ihren Zellen eine Expression der transformierten Nucleinsäure stattfinden kann, so daß eine Pflanze mit verändertem Metabolit-Gehalt erhalten wird.

Zur Modifikation der Eigenschaften des mitochondrialen Transports einer Pflanze kann sowohl die Spezifität des Transportsystems, wobei der Transport neuer Verbindungen ermöglicht wird, als auch der Transportmechanismus verändert werden. Beispielsweise betrifft die Erfindung Modifikationen des mitochondrialen und/oder plastidären Transports in einer Pflanze, im Rahmen derer die Affinität und/oder Substratspezifität eines Transporters der inneren Mitochondrienmembran, insbesondere eines MCF-Transportermoleküls, dahingehend verändert wird, dass es zu einem effizienteren Transport in die oder aus den Mitochondrien kommt. Dies kann geschehen, indem zum Beispiel eine Überexpression des MCF-Transportermoleküls bewirkt wird, zum Beispiel durch Einführen mehrerer Genkopien oder von Konstrukten, die die erfindungsgemäße isolierte Nucleinsäuresequenz unter Kontrolle eines starken konstitutiv oder gewebe- und/oder zeitspezifisch exprimierenden Promoters enthalten. Ebenso können Modifikationen des mitochondrialen Transports durch Repression, Suppression und/oder Cosuppression endogener MCF-Gene erzielt werden, wodurch es zum Beispiel zu gezielten Akkumulationen bestimmter Substanzen inner- oder außerhalb der Mitochondrien beziehungsweise Plastiden kommt. Derartige die endogen vorhandene MCF-Aktivität hemmende Effekte können erfindungsgemäß durch Transformation von Pflanzenzellen mit Antisinnkonstrukten und/oder Integration mehrerer Sinn-Konstrukte, um einen Cosupressionseffekt zu erhalten, und/oder mittels eines Knockout-Ansatzes erzielt werden. Selbstverständlich können auch endogen vorhandene Gene der Transporter der inneren Mitochondrienmembran mittels der erfindungsgemäßen Nucleinsäuren in ihrer Aktivität direkt gehemmt werden, beispielsweise durch Integration in die endogen vorhandene Nucleinsäure mittels homologer Rekombination.

Die vorliegende Erfindung betrifft also auch die Verwendung der erfindungsgemäßen Nucleinsäuren zur Modifikation des mitochondrialen und/oder plastidären Transports, insbesondere an der inneren Mitochondrienmembran von Pflanzen oder an der inneren Plastidenmembran von Pflanzen.

Die Erfindung stellt in bevorzugter Ausführungsform Verfahren zur Verwendung der vorgenannten Nucleinsäuren zur Verfügung, gemäß derer insbesondere der Aminosäuretransport an der inneren Mitochondrienmembran modifiziert wird.

Insbesondere betrifft die Erfindung Verfahren zur Modifikation des mitochondrialen Arginin- und Ornithin-Transports. Die Aminosäure Arginin stellt in den Samen und Speicherorganen vieler Pflanzen die Hauptspeicherform für Stickstoff dar. Während der Keimung wird das in Speicherproteine eingebaute oder als freie Aminosäure vorliegende Arginin durch das Enzym Arginase zunächst in Ornithin und Harnstoff gespalten. Der so entstandene Harnstoff wird dann von Urease zu Ammonium und CO₂ abgebaut. Das freigesetzte Ammonium kann dann in die während der Keimung besonders wichtige Aminosäure-Biosynthese einfließen. Die beiden Enzyme Arginase und Urease sind in pflanzlichen Zellen in der Matrix der Mitochondrien lokalisiert, so dass das im Cytosol vorliegende Arginin zunächst durch die innere Mitochondrienmembran transportiert werden muss.

Eine besonders bevorzugte Ausführungsform der Erfindung sieht daher die Überexpression oder ektopische Expression des erfindungsgemäßen pflanzlichen mitochondrialen Arginin/Ornithin-Transporters unter Kontrolle verschiedener Promotoren, zum Beispiel CaMV-35S oder samenspezifische Promotoren, vor, um die Effizienz der Stickstoffmobilisierung durch den Arginin-Abbau zu erhöhen und so das Keimungsverhalten zu verbessern.

Ornithin ist eine Vorstufe für die Synthese von Alkaloiden wie Nikotin, Atropin und Kokain. Durch Veränderung des Ornithin-Pools kann daher eine Veränderung des Alkaloid-Gehalts erreicht werden.

Eine weitere besonders bevorzugte Ausführungsform der Erfindung sieht daher die Überexpression oder ektopische Expression und anti-sense-Inhibition des pflanzlichen Arginin/Ornithin-Transporters zur Veränderung des Alkaloid-Gehalts in Pflanzen vor.

Die erfindungsgemäß verwendeten Nucleinsäuren, insbesondere eine Nucleinsäure mit einer in SEQ ID Nr. 1 dargestellten Sequenz, können darüber hinaus zur Isolierung homologer Sequenzen aus Bakterien, Pilzen, Pflanzen, Tieren und/oder Menschen verwendet werden. Um nach homologen Sequenzen suchen zu können, müssen zunächst Genbanken angelegt werden, beispielsweise genomische Banken oder oder cDNA-Banken. Mit Hilfe einer Sonde, die Teile der vorstehend beschriebenen Nucleinsäuren enthält, können dann aus den Genbanken verwandte Sequenzen isoliert werden. Nach Identifizierung und/oder Isolierung der entsprechenden Gene können DNA- und Aminosäure-Sequenzen bestimmt und die Eigenschaften der von diesen Sequenzen codierten Proteine analysiert werden.

Ferner können die erfindungsgemäß verwendeten Nucleinsäuren verwendet werden, um die Expression eines erfindungsgemäßen Transporters der inneren Mitochondrienmembran, insbesondere eines MCF-Mitglieds, in pro- und/oder eukaryontischen Zellen zu untersuchen. Werden die vorstehend beschriebenen Nucleinsäuren beispielsweise in prokaryontischen Zellen, wie Bakterien, eingeführt, so wird eine von Bakterien translatierbare RNA-Sequenz eines eukaryontischen Transporters der inneren Mitochondrienmembran, insbesondere eines eukaryontischen MCF-Mitglieds, gebildet, die trotz der erheblichen Unterschiede in den Membranstrukturen von Pro- und Eukaryonten in ein funktionales eukaryontisches MCF-Mitglied mit dessen Substratspezifität translatiert wird. Diese Bakterienzellen können daher für Untersuchungen der Eigenschaften eines Transportermoleküls sowie seiner Substrate verwendet werden. Die erfindungsgemäßen Nucleinsäuren können erfindungsgemäß unter der Kontrolle eines regulatorischen Elements in Antisinnorientierung zur Hemmung der Expression eines endogenen Transporters der inneren Mitochondrienmembran, insbesondere eines endogenen MCF-Mitglieds, in pro- und/oder eukaryontischen Zellen verwendet werden. Die Herstellung transgener Nutzpflanzen stellt eine weitere Verwendungsmöglichkeit dieser Nucleinsäuren dar.

Die erfindungsgemäß verwendeten Nucleinsäuren beziehungsweise die von ihnen codierten MCF-Transportermoleküle können darüber hinaus auch für Untersuchungen von Herbiziden verwendet werden. Einige der für Pflanzen essentiellen Transportermoleküle stellen Targets für Herbizide dar, die die Funktion der Transporter inhibieren können. Bevorzugt können insbesondere in Hefe Screeningverfahren durchgeführt werden, um nach Inhibitoren der erfindungsgemäßen MCF-Transporter zu suchen. Im Hefesystem können diese Inhibitoren weiter getestet und gegebenenfalls durch chemische Modifikation optimiert werden. Anschließend kann ein Test dieser Inhibitoren an Pflanzen durchgeführt werden. Die Verwendung der erfindungsgemäßen MCF-Transporter zur Untersuchung von Herbiziden ist insofern von besonderem Interesse, als die mitochondrial carrier-Familie Eukaryonten-spezifisch ist, so dass ein gegen ein MCF-Mitglied gerichtetes Herbizid für Bodenbakte-rien unschädlich sein sollte. Aufgrund der vergleichsweise hohen Divergenz zwischen Protein-Sequenzen der MCFs aus verschiedenen Spezies ist es deshalb möglich, Herbizide mit geringer Toxizität gegenüber nicht-pflanzlichen Organismen zu identifizieren.

Da der Wirkungsort vieler Herbizide in den Plastiden beziehungsweise Mitochondrien liegt, ist ihr Eintransport ein guter Ansatzpunkt für die Entwicklung neuer Resistenzmechanismen. Durch eine erfindungsgemäß vorgesehene gezielte Veränderung der Substratspezifität plastidär lokalisierter erfindungsgemäßer MCF-Mitglieder lassen sich Nutzpflanzen erzeugen, in denen Herbizide ihren Wirkungsort nicht erreichen.

Die folgenden Figuren und Beispiele erläutern die Erfindung. Die Figuren zeigen:

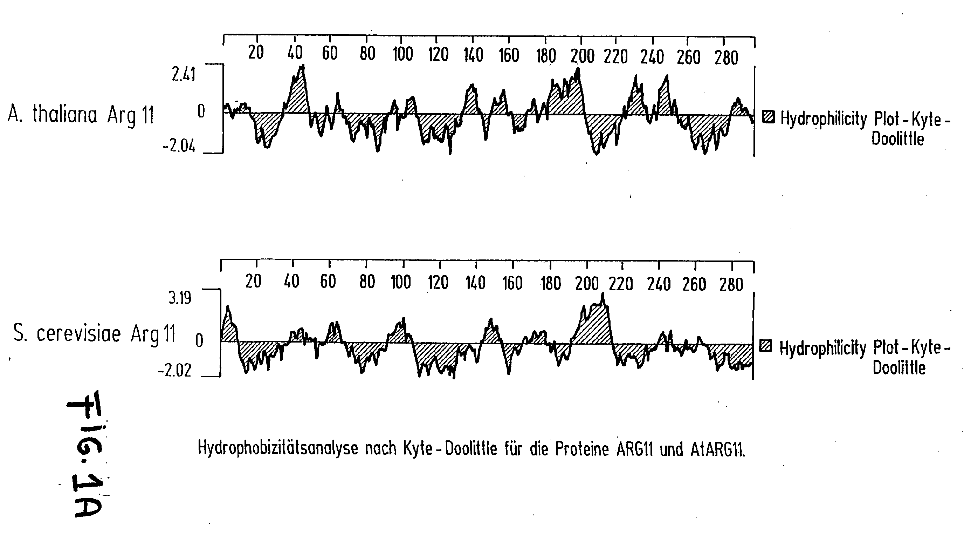

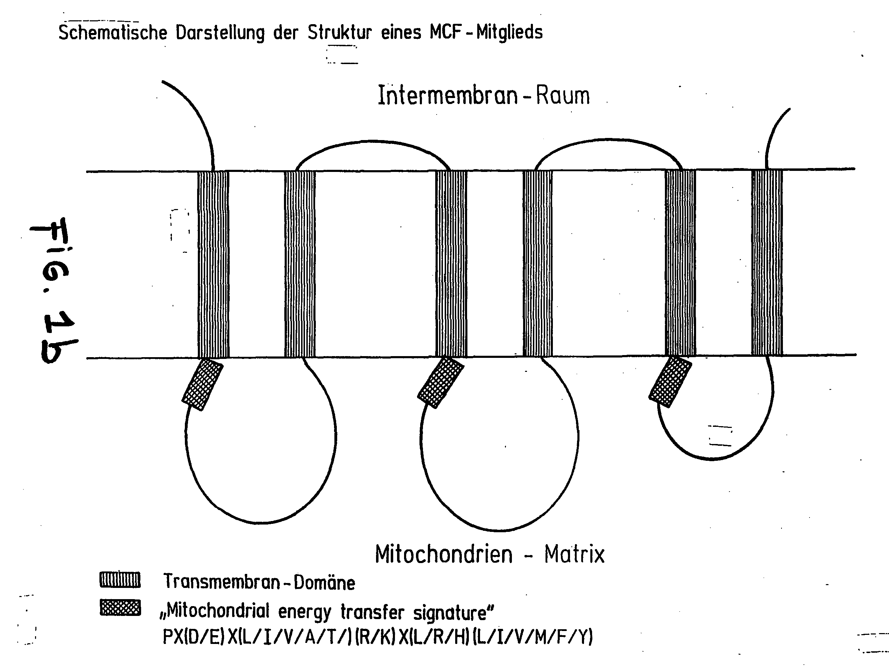

- Figur 1: a) eine Hydrophobizitätsanalyse nach Kyte-Doolittle für die Proteine ARG11 und atARG11 ,
b) eine schematische Darstellung der Struktur eines MCF-Mitglieds am Beispiel von AtARG11,

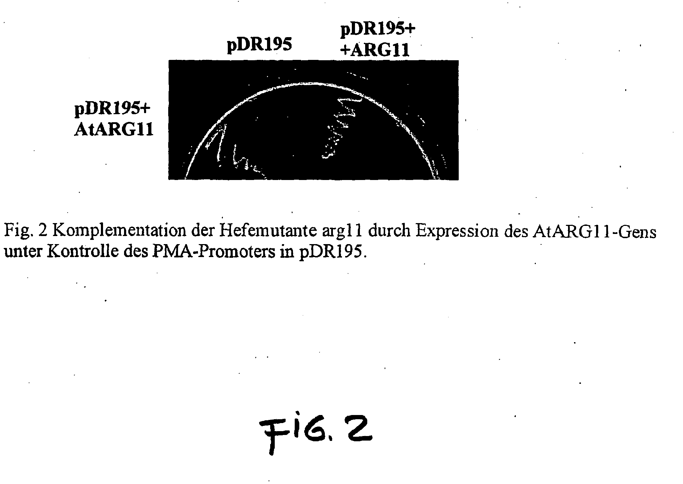

- Figur 2: die Komplementation der Hefemutante arg ARG11 durch Expression des AtARG11-Gens unter Kontrolle des PMA-Promoters,

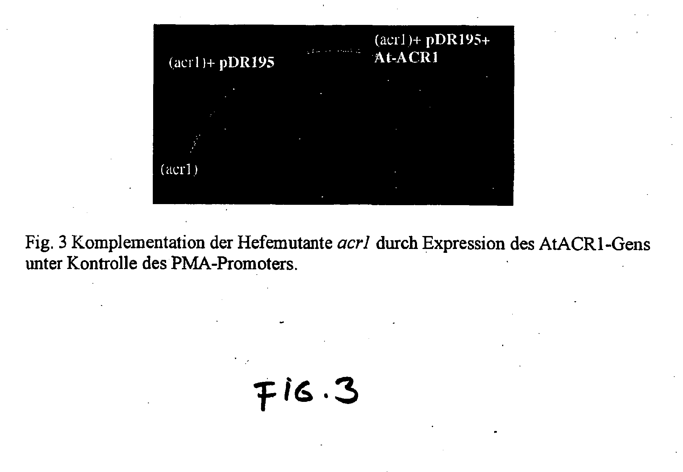

- Figur 3: die Komplementation der Hefemutante acr1 durch Expression des AtACR1-Gens unter Kontrolle des PMA-Promoters.

Das Sequenzprotokoll umfasst:
SEQ ID Nr. 1 zeigt die 891 Nucleotide umfassende DNA-Sequenz des Gens AtARG11 aus *Arabidopsis thaliana*.
SEQ ID Nr. 2 zeigt die davon abgeleitete 296 Aminosäuren umfassende Aminosäuresequenz des erfindungsgemäßen mcf-Proteins.
SEQ ID Nr. 3 zeigt die 930 Nucleotide umfassende cDNA-Sequenz des AtACR1-Gens aus *Arabidopsis thaliana.*
SEQ ID Nr. 4 zeigt die davon abgeleitete 309 Aminosäuren umfassende Aminosäuresequenz eines weiteren erfindungsgemäßen mcf-Proteins.
Die SEQ ID Nr. 5 bis 10 zeigen für die Clonierung verwendete Primer aus *Arabidopsis thaliana.*

### Beispiele:

Allgemeine Verfahren:
a) Clonierungsverfahren: zur Clonierung in *E. coli* und zur Transformation von Hefen wurde der Vektor pDR195 (D. Rentsch et al., FEBS Lett., 370 (1995), 264-268) eingesetzt. Zur Pflanzentransformation wurden die Genkonstrukte in den binären Vektor CHF3, ein Derivat von pPZP212 (P. Hajdukiewicz et al., Plant Mol. Biol., 25 (1994), 989-994) cloniert.
   Zur Lokalisierung des Proteins in Pflanzen wurde der Vektor pSMGFP4 verwendet.
b) Bakterien- und Hefestämme: Für die Amplifikation und Clonierung der verschiedenen Vektoren wurde der *E. coli*-Stamm DH5α verwendet.
   Als Hefestamm wurden 1c1636d (arg11-1, ura3⁻), ein Derivat von MG409 (ura3⁻) (Delforge et al., Eur. J. Biochem., 57 (1975), 231), und G60 (acr1, ura3⁻) ein Derivat von 23344c (ura3⁻) (Grenson et al.) eingesetzt.
c) Transformation von *Agrobacterium tumefaciens:* Der DNA-Transfer in die Agrobakterien erfolgte durch direkte Transformation nach dem Verfahren von Höfgen und Willmitzer (Nucl. Acids Res., 16 (1988), 9877). Die Plasmid-DNA transformierter Agrobakterien wurde nach dem Verfahren von Birnboim und Doly (Nucl. Acids Res., 7 (1979), 1513-1523) isoliert und nach Spaltung mit geeigneten Restriktionsenzymen gelelektrophoretisch analysiert.
d) Pflanzentransformation: Die Pflanzentransformation kann mittels eines Gentransfers, der durch *Agrobacterium* tumefaciens (Stamm C58Cl, pGV2260) vermittelt wird, erfolgen (Deblaere et al., Nuc. Acids Res., 13 (1985), 4777-4788). Die Transformation von *A. thaliana* erfolgt beispielsweise mittels Vakuum-Infiltration (modifiziert nach Bechtold et al. (Comptes Rendus de l'Academie des Sciences Serie III, Sciences de la Vie, 316 (1993), 1194-1199). Töpfe (Durchmesser 10 cm) werden mit Erde gefüllt und anschließend mit einem Fliegennetz überspannt. Auf diesem Netz werden A. *thaliana*-Samen ausgesät. Sechs bis acht Wochen nach dem Aussäen wurden die Pflanzen für die Vakuum-Infiltration benutzt. Zur Vakuum-Infiltration wurden von den entsprechenden Agrobacterium-Stämmen 2 x 1 Liter Kulturen in YEB-Medium angezogen, das Antibiotika enthielt (50 µg/ml Kanamycin und 100 µg/ml Rifampicin) bei 28°C angezogen. Bei einer OD₆₀₀ von 1,5 werden die Zellen bei 3000 g geerntet und in 600 ml Infiltrationsmedium (0,5 x MS-Medium (Sigma), 5% Saccharose, 44 µM Benzylaminopurin) resuspendiert. Die Bakteriensuspension wird in 250 ml Weckgläser gefüllt und in einen Exsikkator gestellt- Die A. *thaliana-*Pflanzen werden "kopfüber" in die Baktierensuspension getaucht, dann wird ein fünfminütiges Vakuum angelegt. Nach 3-4 Wochen werden die Samen dieser Pflanze geerntet. Zur Oberflächensterilisation werden die Samen 10 Minuten in 4% Natriumhypochlorid, 0,02% Triton geschüttelt, bei 1500 g abzentrifugiert, viermal mit sterilem Wasser gewaschen und in 3 ml 0,05% Agarose (pro 5000 Samen) resuspendiert. Die Samen-Agarose-Lösung wird auf MSS Medium (1 x MS, 1% Saccharose, 0,8% Agarose,
   50 µ/ml Kanamycin, pH 5,8) ausgebreitet (Platten mit 13,5 cm Durchmesser für 5000 Samen). Zur Reduzierung des Feuchtigkeitsverlustes werden die Platten mit Parafilm® verschlossen. Die Kanamycin-resistenten Pflanzen werden in Erde umgesetzt. Samen dieser Pflanzen werden geerntet und analysiert.

### Beispiel 1:

### Clonierung des Arginin/Ornithin-Transportergens aus Arabidopsis thaliana

Das AtARG11-Gen wurde mit Hilfe des PCR-Verfahrens aus einer cDNA-Genbank von *Arabidopsis thaliana* mit den Primern AtARG11-f (5'-agcctcgagatggatttctggccggagtttatg-3', SEQ ID Nr. 5) und AtARG11-r (5'-ttcggatcctcaatctcctgtgacaatatc-3', SEQ ID Nr. 6) amplifiziert. Der Primer AtARG11-f enthält eine XhoI-Spaltstelle und der Primer AtARG11-r eine BamHI-Spaltstelle. Das PCR-Produkt wurde mit den Restriktionsenzymen BamHI und XhoI gespalten und in das Plasmid pDR 195, das ebenfalls mit den Restriktionsenzymen BamHI und XhoI gespalten worden war, ligiert. Anschließend wurde das Plasmid in *E. coli-*Zellen transformiert.

Die Insertionen in den Plasmiden wurden sequenziert. Eine cDNA-Sequenz mit 891 Nucleotiden konnte ermittelt werden (SEQ ID Nr. 1). SEQ ID Nr. 2 zeigt die dazugehörige Aminosäuresequenz des erfindungsgemäßen Arginin-Ornithin-Transportermoleküls.

Etwa 1 µg des Plasmids wurden nach dem Verfahren von Gietz (Gietz et al., Yeast 11 (1995), 355-360) in den Hefestamm 1c1636d transformiert. Anschließend wurden Hefe-Transformanten selektiert, die auf Minimalmedium ohne Arginin wachsen konnten.

### Beispiel 2:

### Clonierung des Succinat-Transportergens aus Arabidopsis thaliana

Das AtACR1-Gen wurde mit Hilfe des PCR-Verfahrens aus einer cDNA-Genbank von *Arabidopsis thaliana* mit dem Primer AtACR1-f (5'-acgctcgagatggcgacgagaacggaatc-3', SEQ ID Nr. 7), der eine Spaltstelle für das Restriktionsenzym XhoI enthält, und dem Primer AtACR1-r (5'-acggcggccgcctataaaggagcattccgaag-3', SEQ ID Nr. 8), der eine NotI-Spaltstelle enthält, isoliert.

Das PCR-Produkt wurde mit den Restriktionsenzymen XhoI und NotI gespalten und in das Plasmid pDR 195, das ebenfalls mit den Restriktionsenzymen XhoI und NotI gespalten worden war, ligiert. Anschließend wurden E. coli-Zellen mit dem Plasmid transformiert.

Das Insert des Plasmides wurde sequenziert. Es konnte eine 930 Nucleotide umfassende cDNA-Sequenz ermittelt werden (SEQ ID Nr. 3). Die Aminosäuresequenz des von dieser DNA-Sequenz codierten Succinat-Transporters ist in SEQ ID Nr. 4 gezeigt und umfasst 309 Aminosäuren.

Etwa 1 µg des Plasmids nach dem Verfahren von Gietz (Gietz et al., Yeast 11 (1995), 355-360) wurden in den Hefestamm G60 transformiert. Anschließend wurden Hefe-Transformanten selektiert, die auf Minimalmedium mit Ethanol als einziger Kohlenstoffquelle wachsen konnten.

### Beispiel 3:

### Clonierung der AtARG11- und AtACR1-Gene in den Vektor CHF3

Das AtACR1-Gen und das AtARG11-Gen wurden in den Vektor CHF3 cloniert, um in Pflanzen eine Überexpression zu erreichen. Die Gene wurden aus den entsprechenden pDR195-Konstrukten mit BamHl und Xhol herausgeschnitten und in den mit BamHl und Smal gespaltenen Vektor CHF3 ligiert (CHF3-ARG11). Die erzeugten Plasmide wurden in Agrobakterium tumefaciens transformiert und die resultierenden Bakterienstämme wurden zur Transformation von Arabidopsis verwendet.

Zur Lokalisierung in Pflanzen wurde das AtARG11-Gen mittels PCR aus einer cDNA-Genbank *von Arabidopsis thaliana* mit den Primern AtARG11-GFP-f (5'-ttcggat-ccatggatttctggccggagtttatg-3', SEQ ID Nr. 9) und AtARG11-GFP-r(5'aatcccgggatctcctgtgacaatatctggg-3', SED IQ Nr. 10) ämplifiziert. Der Primer AtARG11-GFP-f enthält eine BamHI-Spaltstelle und der Primer AtARG11-GFP-r eine Smal-Schnittstelle. Das mit BamHI und Smal gespaltene PCR-Produkt wurde in den bVektor pSMGFP4 ligiert, der ebenfalls mit BamHI und SmaI gespalten worden war. Das Genprodukt wurde mit Hilfe von Fluoreszenzmikroskopie subzellulär lokalisiert.

## Patentansprüche

1. Verfahren zur Veränderung des Metabolitgehaltes von Pflanzen durch Modifikation der Transportereigenschaften der inneren Mitochondrienmembran einer Pflanze, eines Pflanzenteils oder einer Pflanzenzelle, umfassend
- das Einbringen einer Nucleinsäure in eine Pflanzenzelle und
- gegebenenfalls das Regenerieren einer ganzen Pflanze daraus,
wobei die Nucleinsäure ausgewählt ist aus der Gruppe bestehend aus:
a) einer Nucleinsäure, die erhältlich ist durch Komplementierung MCF-defizienter Wirtszellen, die einen Defekt in dem Transportermolekül aufweisen, mit Nucleinsäuresequenzen einer pflanzlichen Genbank und durch Selektion MCF-positiver Wirtszellen;
b) einer Nucleinsäure mit einer in SEQ ID Nr. 1 dargestellten Nucleotidsequenz;
c) einer Nucleinsäure mit einer Sequenz, die ein Protein mit einer in SEQ ID Nr. 2 dargestellten Sequenz codiert;
d) einer Nucleinsäure, die zu einer Nucleinsäure nach a) bis c) komplementär ist;
e) einer Nucleinsäure, die durch Substitution, Addition, Inversion und/oder Deletion einer oder mehrerer Basen einer Nucleinsäure nach a) bis d) erhältlich ist und in einer Wirtszelle in Antisinnorientierung zu einem Promoter die Expression eines pflanzlichen Arginin/Ornithin-Transportermoleküls hemmen kann;
f) einer Nucleinsäure, die aufgrund der Degeneration des genetischen Codes mit einer Nucleinsäure nach a) bis e) hybridisiert; und
g) einem Fragment einer Nucleinsäure nach a), b), c), d), e) oder f), welches mindestens 10 Nucleotide, vorzugsweise mindestens 50 Nucleotide, besonders bevorzugt mindestens 200 Nucleotide umfasst und in einer Wirtszelle in Antisinnorientierüng zu einem Promoter die Expression eines pflanzlichen Arginin/Ornithin-Transportermoleküls hemmen kann.

2. Verfahren nach Anspruch 1, wobei ein die Nucleinsäure enthaltendes Konstrukt, worin die Nucleinsäure unter der Kontrolle von Expressions-Regulationselementen steht, in eine Pflanzenzelle eingebracht wird.

3. Verfahren nach Anspruch 2, wobei sich die Nucleinsäure in dem Konstrukt Antisinn-Orientierung zu dem Regulationselement befindet.

4. Verfahren nach Anspruch 2 oder 3, wobei das Konstrukt in Form eines Plasmids vorliegt.

5. Verfahren zur Isolierung von mindestens einem Gen, codierend einen pflanzlichen Arginin/Ornithin-Transporter der inneren Mitochondrienmembran, aus einer Genbank, wobei die MCF-defiziente Hefezelle *arg11* mit Nucleinsäuresequenzen aus einer pflanzlichen Genbank transformiert und eine positive Selektion durchgeführt wird.

6. Verwendung einer unter der Kontrolle eines Regulationselements in Antisinn-Orientierung befindlichen Nucleinsäure, ausgewählt aus der Gruppe bestehend aus:
a) einer Nucleinsäure, die erhältlich ist durch Komplementierung MCF-defizienter Wirtszellen, die einen Defekt in dem Transportermolekül aufweisen, mit Nucleinsäuresequenzen einer pflanzlichen Genbank und durch Selektion MCF-positiver Wirtszellen;
b) einer Nucleinsäure mit einer in SEQ ID Nr. 1 dargestellten Nucleotidsequenz;
c) einer Nucleinsäure mit einer Sequenz, die ein Protein mit einer in SEQ ID Nr. 2 dargestellten Sequenz codiert;
d) einer Nucleinsäure, die zu einer Nucleinsäure nach a) bis c) komplementär ist;
e) einer Nucleinsäure, die durch Substitution, Addition, Inversion und/oder Deletion einer oder mehrerer Basen einer Nucleinsäure nach a) bis d) erhältlich ist und in einer Wirtszelle in Antisinnorientierung zu einem Promoter die Expression eines pflanzlichen Arginin/Ornithin-Transportermoleküls hemmen kann;
f) einer Nucleinsäure, die aufgrund der Degeneration des genetischen Codes mit einer Nucleinsäure nach a) bis e) hybridisiert; und
g) einem Fragment einer Nucleinsäure nach a), b), c), d), e) oder f), welches mindestens 10 Nucleotide, vorzugsweise mindestens 50 Nucleotide, besonders bevorzugt mindestens 200 Nucleotide umfasst und in einer Wirtszelle in Antisinnorientierung zu einem Promoter die Expression eines pflanzlichen Arginin/Ornithin-Transportermoleküls hemmen kann,
zur Hemmung der Expression eines endogenen MCF-Mitglieds der inneren Mitochondrienmembran pflanzlicher Zellen.

7. Verwendung einer Nucleinsäure, ausgewählt aus der Gruppe bestehend aus:
a) einer Nucleinsäure, die erhältlich ist durch Komplementierung MCF-defizienter Wirtszellen, die einen Defekt in dem Transportermolekül aufweisen, mit Nucleinsäuresequenzen einer pflanzlichen Genbank und durch Selektion MCF-positiver Wirtszellen;
b) einer Nucleinsäure mit einer in SEQ ID Nr. 1 dargestellten Nucleotidsequenz;
c) einer Nucleinsäure mit einer Sequenz, die ein Protein mit einer in SEQ ID Nr. 2 dargestellten Sequenz codiert;
d) einer Nucleinsäure, die zu einer Nucleinsäure nach a) bis c) komplementär ist;
e) einer Nucleinsäure, die durch Substitution, Addition, Inversion und/oder Deletion einer oder mehrerer Basen einer Nucleinsäure nach a) bis d) erhältlich ist und in einer Wirtszelle in Antisinnorientierung zu einem Promoter die Expression eines pflanzlichen Arginin/Ornithin-Transportermoleküls hemmen kann;
f) einer Nucleinsäure, die aufgrund der Degeneration des genetischen Codes mit einer Nucleinsäure nach a) bis e) hybridisiert; und
g) einem Fragment einer Nucleinsäure nach a), b), c), d), e) oder f), welches mindestens 10 Nucleotide, vorzugsweise mindestens 50 Nucleotide, besonders bevorzugt mindestens 200 Nucleotide umfasst und in einer Wirtszelle in Antisinnorientierung zu einem Promoter die Expression eines pflanzlichen Arginin/Ornithin-Transportermoleküls hemmen kann,
zur Herstellung transgener Nutzpflanzen mit veränderten mitochondrialen Transporteigenschaften

8. Verwendung einer Nucleinsäure, ausgewählt aus der Gruppe bestehend aus:
a) einer Nucleinsäure, die erhältlich ist durch Komplementierung MCF-defizienter Wirtszellen, die einen Defekt in dem Transportermolekül aufweisen, mit Nucleinsäuresequenzen einer pflanzlichen Genbank und durch Selektion MCF-positiver Wirtszellen;
b) einer Nucleinsäure mit einer in SEQ ID Nr. 1 dargestellten Nucleotidsequenz;
c) einer Nucleinsäure mit einer Sequenz, die ein Protein mit einer in SEQ ID Nr. 2 dargestellten Sequenz codiert;
d) einer Nucleinsäure, die zu einer Nucleinsäure nach a) bis c) komplementär ist;
e) einer Nucleinsäure, die durch Substitution, Addition, Inversion und/oder Deletion einer oder mehrerer Basen einer Nucleinsäure nach a) bis d) erhältlich ist und in einer Wirtszelle in Antisinnorientierung zu einem Promoter die Expression eines pflanzlichen Arginin/Ornithin-Transportermoleküls hemmen kann;
f) einer Nucleinsäure, die aufgrund der Degeneration des genetischen Codes mit einer Nucleinsäure nach a) bis e) hybridisiert; und
g) einem Fragment einer Nucleinsäure nach a), b), c), d), e) oder f), welches mindestens 10 Nucleotide, vorzugsweise mindestens 50 Nucleotide, besonders bevorzugt mindestens 200 Nucleotide umfasst und in einer Wirtszelle in Antisinnorientierung zu einem Promoter die Expression eines pflanzlichen Arginin/Ornithin-Transportermoleküls hemmen kann,
zur Identifizierung von Inhibitoren des durch Transporter der inneren Mitochondrienmembran vermittelten Stofftransports in pflanzlichen Mitochondrien.

## Claims

1. Method for modifying the metabolite content of plants by modification of the transporter properties of the inner mitochondrial membrane of a plant, a plant part, or a plant cell, comprising
- the insertion of a nucleic acid into a plant cell, and
- the regeneration of a complete plant from that plant cell, if applicable,
wherein the nucleic acid is selected from the group consisting of:
a) a nucleic acid obtainable by complementation of MCF deficient host cells having a defect in the transporter molecule with nucleic acid sequences from a plant gene bank, and by selection of MCF positive host cells;
b) a nucleic acid with a nucleotide sequence shown in SEQ ID No. 1;
c) a nucleic acid with a sequence encoding a protein with a sequence shown in SEQ ID No. 2;
d) a nucleic acid that is complementary to a nucleic acid according to a) to c);
e) a nucleic acid obtainable by substitution, addition, inversion, and/or deletion of one or more bases of a nucleic acid according to a) to d) and adapted to inhibit the expression of a plant arginine/ornithine transporter molecule in a host cell in antisense orientation to a promoter;
f) a nucleic acid that hybridizes with a nucleic acid according to a) to e) because of the degeneration of the genetic code; and
g) a fragment of a nucleic acid according to a), b), c), d), e) or f), comprising at least 10 nucleotides, preferably at least 50 nucleotides, in particular at least 200 nucleotides, and adapted to inhibit the expression of a plant arginine/ornithine transporter molecule in a host cell in antisense orientation to a promoter.

2. Method according to claim 1, wherein a construct that includes the nucleic acid, which is under the control of expression regulatory elements, is inserted into a plant cell.

3. Method according to claim 2, wherein the nucleic acid is located in the construct in antisense orientation to the regulatory element.

4. Method according to claim 2 or 3, wherein the construct is present in the form of a plasmid.

5. Method for isolating at least one gene encoding a plant arginine/ornithine transporter of the inner mitochondrial membrane from a gene bank, wherein the MCF deficient yeast cell *arg11* is transformed with nucleic acid sequences from a plant gene bank and a positive selection is executed.

6. Use of a nucleic acid under the control of a regulatory element in antisense orientation, selected from the group consisting of:
a) a nucleic acid obtainable by complementation of MCF deficient host cells having a defect in the transporter molecule with nucleic acid sequences from a plant gene bank, and by selection of MCF positive host cells;
b) a nucleic acid with a nucleotide sequence shown in SEQ ID No. 1;
c) a nucleic acid with a sequence encoding a protein with a sequence shown in SEQ ID No. 2;
d) a nucleic acid that is complementary to a nucleic acid according to a) to c);
e) a nucleic acid obtainable by substitution, addition, inversion, and/or deletion of one or more bases of a nucleic acid according to a) to d) and adapted to inhibit the expression of a plant arginine/ornithine transporter molecule in a host cell in antisense orientation to a promoter:
f) a nucleic acid that hybridizes with a nucleic acid according to a) to e) because of the degeneration of the genetic code; and
g) a fragment of a nucleic acid according to a), b), c), d), e) or f), comprising at least 10 nucleotides, preferably at least 50 nucleotides, in particular at least 200 nucleotides, and adapted to inhibit the expression of a plant arginine/ornithine transporter molecule in a host cell in antisense orientation to a promoter,
for inhibiting the expression of an endogenous MCF member of the inner mitochondrial membrane of plant cells.

7. Use of a nucleic acid, selected from the group consisting of:
a) a nucleic acid obtainable by complementation of MCF deficient host cells having a defect in the transporter molecule with nucleic acid sequences from a plant gene bank, and by selection of MCF positive host cells;
b) a nucleic acid with a nucleotide sequence shown in SEQ ID No. 1;
c) a nucleic acid with a sequence encoding a protein with a sequence shown in SEQ ID No. 2;
d) a nucleic acid that is complementary to a nucleic acid according to a) to c);
e) a nucleic acid obtainable by substitution, addition, inversion, and/or deletion of one or more bases of a nucleic acid according to a) to d) and adapted to inhibit the expression of a plant arginine/ornithine transporter molecule in a host cell in antisense orientation to a promoter;
f) a nucleic acid that because of the degeneration of the genetic code hybridizes with a nucleic acid according to a) to e); and
g) a fragment of a nucleic acid according to a), b), c), d), e) or f), comprising at least 10 nucleotides, preferably at least 50 nucleotides, in particular at least 200 nucleotides, and adapted to inhibit the expression of a plant arginine/ornithine transporter molecule in a host cell in antisense orientation to a promoter,
for producing transgenic crop plants with modified mitochondrial transport properties.

8. Use of a nucleic acid, selected from the group consisting of:
a) a nucleic acid obtainable by complementation of MCF deficient host cells having a defect in the transporter molecule with nucleic acid sequences from a plant gene bank, and by selection of MCF positive host cells;
b) a nucleic acid with a nucleotide sequence shown in SEQ ID No. 1;
c) a nucleic acid with a sequence encoding a protein with a sequence shown in SEQ ID No. 2;
d) a nucleic acid that is complementary to a nucleic acid according to a) to c);
e) a nucleic acid obtainable by substitution, addition, inversion, and/or deletion of one or more bases of a nucleic acid according to a) to d) and adapted to inhibit the expression of a plant arginine/ornithine transporter molecule in a host cell in antisense orientation to a promoter;
f) a nucleic acid that because of the degeneration of the genetic code hybridizes with a nucleic acid according to a) to e); and
g) a fragment of a nucleic acid according to a), b), c), d), e) or f), comprising at least 10 nucleotides, preferably at least 50 nucleotides, in particular at least 200 nucleotides, and adapted to inhibit the expression of a plant arginine/ornithine transporter molecule in a host cell in antisense orientation to a promoter,
for identifying inhibitors of the substance transport mediated by transporters of the inner mitochondria membrane in plant mitochondria.

## Revendications

1. Procédé pour modifier le contenu en métabolites de plantes par modification des propriétés de transporteur de la membrane mitochondriale interne d'une plante, d'une partie d'une plante ou d'une cellule d'une plante, comprenant
- l'insertion d'un acide nucléique dans une cellule d'une plante ; et,
- le cas échéant, la régénération d'une plante complète à partir de cette cellule,
dans lequel l'acide nucléique est choisi du groupe constitué de :
a) un acide nucléique obtenable par complémentation de cellules hôtes MCFdéficientes (MCF = famille de transporteurs mitochondriaux) présentant un défaut dans la molécule transporteur avec des séquences d'acides nucléiques d'une banque de gènes végétaux, et par sélection de cellules hôtes MCF positives ;
b) un acide nucléique avec une séquence de nucléotides représentée dans SEQ ID no. 1 ;
c) un acide nucléique avec une séquence codant une protéine avec une séquence représentée dans SEQ ID no. 2 ;
d) un acide nucléique complémentaire à un acide nucléique selon a) à c) ;
e) un acide nucléique obtenable par substitution, addition, inversion et/ou délétion d'une ou plusieurs bases d'un acide nucléique selon a) à d) et adapté pour inhiber l'expression d'une molécule transporteur végétale arginine/ornithine dans une cellule hôte en orientation antisens par rapport à un promoteur ;
f) un acide nucléique hybridant avec un acide nucléique selon a) à e) grâce à la dégénération du code génétique ; et
g) un fragment d'un acide nucléique selon a), b), c), d), e) ou f), comprenant au moins 10 nucléotides, de préférence au moins 50 nucléotides, de préférence particulière au moins 200 nucléotides, et adapté pour inhiber l'expression d'une molécule transporteur végétale arginine/ornithine dans une cellule hôte en orientation antisens par rapport à un promoteur.

2. Procédé selon la revendication 1, dans lequel une structure qui contient l'acide nucléique et dans laquelle l'acide nucléique est sous le contrôle d'éléments régulateurs d'expression est insérée dans une cellule d'une plante.

3. Procédé selon la revendication 2, dans lequel l'acide nucléique contenu dans la structure se trouve en orientation antisens par rapport à l'élément régulateur.

4. Procédé selon la revendication 2 ou 3, dans lequel la structure est présente sous forme d'un plasmide.

5. Procédé pour l'isolation d'au moins un gène codant un transporteur végétal arginine/ornithine de la membrane mitochondriale interne d'une banque de gènes, dans lequel la cellule de levure MCF déficiente *arg11* est transformée avec des séquences d'acides nucléiques d'une banque de gènes végétaux et une sélection positive est effectuée.

6. Utilisation d'un acide nucléique qui se trouve sous le contrôle d'un élément régulateur en orientation antisens, choisi du groupe constitué de :
a) un acide nucléique obtenable par complémentation de cellules hôtes MCFdéficientes présentant un défaut dans la molécule transporteur avec des séquences d'acides nucléiques d'une banque de gènes végétaux, et par sélection de cellules hôtes MCF positives ;
b) un acide nucléique avec une séquence de nucléotides représentée dans SEQ ID no. 1 ;
c) un acide nucléique avec une séquence codant une protéine avec une séquence représentée dans SEQ ID no. 2 ;
d) un acide nucléique complémentaire à un acide nucléique selon a) à c);
e) un acide nucléique obtenable par substitution, addition, inversion et/ou délétion d'une ou plusieurs bases d'un acide nucléique selon a) à d) et adapté pour inhiber l'expression d'une molécule transporteur végétale arginine/ornithine dans une cellule hôte en orientation antisens par rapport à un promoteur ;
f) un acide nucléique hybridant avec un acide nucléique selon a) à e) grâce à la dégénération du code génétique ; et
g) un fragment d'un acide nucléique selon a), b), c), d), e) ou f), comprenant au moins 10 nucléotides, de préférence au moins 50 nucléotides, de préférence particulière au moins 200 nucléotides, et adapté pour inhiber l'expression d'une molécule transporteur végétale arginine/ornithine dans une cellule hôte en orientation antisens par rapport à un promoteur,
pour l'inhibition de l'expression d'un membre endogène MCF de la membrane mitochondriale interne de cellules végétales.

7. Utilisation d'un acide nucléique, choisi du groupe constitué de :
a) un acide nucléique obtenable par complémentation de cellules hôtes MCFdéficientes présentant un défaut dans la molécule transporteur avec des séquences d'acides nucléiques d'une banque de gènes végétaux, et par sélection de cellules hôtes MCF positives ;
b) un acide nucléique avec une séquence de nucléotides représentée dans SEQ ID no. 1 ;
c) un acide nucléique avec une séquence codant une protéine avec une séquence représentée dans SEQ ID no. 2 ;
d) un acide nucléique complémentaire à un acide nucléique selon a) à c) ;
e) un acide nucléique obtenable par substitution, addition, inversion et/ou délétion d'une ou plusieurs bases d'un acide nucléique selon a) à d) et adapté pour inhiber l'expression d'une molécule transporteur végétale arginine/ornithine dans une cellule hôte en orientation antisens par rapport à un promoteur ;
f) un acide nucléique hybridant avec un acide nucléique selon a) à e) grâce à la dégénération du code génétique ; et
g) un fragment d'un acide nucléique selon a), b), c), d), e) ou f), comprenant au moins 10 nucléotides, de préférence au moins 50 nucléotides, de préférence particulière au moins 200 nucléotides, et adapté pour inhiber l'expression d'une molécule transporteur végétale arginine/ornithine dans une cellule hôte en orientation antisens par rapport à un promoteur,
pour la production de plantes utiles transgéniques avec des propriétés de transport mitochondrial modifiées.

8. Utilisation d'un acide nucléique, choisi du groupe constitué de :
a) un acide nucléique obtenable par complémentation de cellules hôtes MCFdéficientes présentant un défaut dans la molécule transporteur avec des séquences d'acides nucléiques d'une banque de gènes végétaux, et par sélection de cellules hôtes MCF positives ;
b) un acide nucléique avec une séquence de nucléotides représentée dans SEQ ID no. 1 ;
c) un acide nucléique avec une séquence codant une protéine avec une séquence représentée dans SEQ ID no. 2 ;
d) un acide nucléique complémentaire à un acide nucléique selon a) à c) ;
e) un acide nucléique obtenable par substitution, addition, inversion et/ou délétion d'une ou plusieurs bases d'un acide nucléique selon a) à d) et adapté pour inhiber l'expression d'une molécule transporteur végétale arginine/ornithine dans une cellule hôte en orientation antisens par rapport à un promoteur ;
f) un acide nucléique hybridant avec un acide nucléique selon a) à e) grâce à la dégénération du code génétique ; et
g) un fragment d'un acide nucléique selon a), b), c) d), e) ou f), comprenant au moins 10 nucléotides, de préférence au moins 50 nucléotides, de préférence particulière au moins 200 nucléotides, et adaptée pour inhiber l'expression d'une molécule transporteur arginine/ornithine végétale dans une cellule hôte dans une direction antisens par rapport à un promoteur,
pour l'identification d'inhibiteurs du transport de substances par l'intermédiaire de transporteurs de la membrane mitochondriale interne dans des mitochondries végétales.
